# EUROPEAN PATENT SPECIFICATION

(11) **EP 0 906 433 B1**
(45) Date of publication and mention of the grant of the patent: **01.06.2005**
(21) Application number: 97916257.5
(22) Date of filing: 17.03.1997
(51) Int. Cl.: C12N 15/55, C12N 9/16, A61K 38/46, C12Q 1/42, C07K 16/40, C12Q 1/68

(54) **PROTEIN TYROSINE PHOSPHATASES OF HEMATOPOIETIC CELLS**
PROTEIN TYROSIN-PHOSPHATASEN VON HEMATOPOIETISCHEN ZELLEN
PROTEINE TYROSINE PHOSPHATASES DE CELLULES HEMATOPOIETIQUES

(30) Priority: 22.03.1996 US 620526
(43) Date of publication of application: 07.04.1999
(73) Proprietor: Genentech, Inc., South San Francisco, CA 94080-4990 (US)
(72) Inventor: LASKY, Laurence, A., Sausalito, CA 94965 (US); CHENG, Jill, Burlingame, CA 94010 (US)
(74) Representative: Pilkington, Stephanie Joan
(86) International application number: PCT/US1997/005278
(87) International publication number: WO 1997/035019

(56) References cited:
- WO-A-91/13989
- BLOOD, vol. 78, 1 November 1991, pages 2222-2228, XP002034263 YI, T. ET AL.: "Identification of novel protein tyrosine phosphatases of hematopoietic cells by polymerase chain reaction amplification"
- BLOOD, vol. 86, 15 December 1995, pages 4454-4467, XP000676765 FENNIE, C. ET AL.: "CD34+ endothelial cell lines derive from murine yolk sac induce the proliferation and differentiation of yolk sac CD34+ hematopoietic progenitors" cited in the application
- MOLECULAR AND CELLULAR BIOLOGY, vol. 14, July 1994, WASHINGTON US, pages 4938-4946, XP000676778 FLORES, E. ET AL.: "Nuclear localization of the PEP protein tyrosine phosphatase" cited in the application
- CELL, vol. 73, 2 July 1993, NA US, pages 1445-1454, XP002034264 SHULTZ, L. ET AL.: "Mutations at the murine Motheaten locus are within the hematopoietic cell protein-tyrosine phosphatase (Hcph) gene." cited in the application
- JOURNAL OF BIOLOGICAL CHEMISTRY, vol. 268, 25 March 1993, MD US, pages 6622-6628, XP002034265 YANG, Q. ET AL.: "Cloning and expression of PTP-PEST" cited in the application
- BLOOD, (1996 AUG 15) 88 (4) 1156-67., XP002034266 CHENG, J. ET AL.: "A novel protein tyrosine phosphatase expressed in lin(lo)CD34(hi)Sca(hi) hematopoietic progenitor cells."
- ONCOGENE , vol. 13, November 1996, pages 2275-2279, XP002034272 KIM, Y. ET AL.: "Characterization of the PEST family protein tyrosine phosphatase BDP1"
- BLOOD, (15 DEC 1996) VOL. 88, NO. 12, PP. 4510-4525., XP002034267 DOSIL, M. ET AL.: "Cloning and characterization of fetal liver phosphatase 1, a nuclear protein tyrosine phosphatase isolated from hematopoietic stem cells"

### SEQUENCE LISTING

(1) GENERAL INFORMATION:
   (i) APPLICANT: Genentech, Inc.
   (ii) TITLE OF INVENTION: Protein Tyrosine Phosphatases
   (iii) NUMBER OF SEQUENCES: 23
   (iv) CORRESPONDENCE ADDRESS:
      (A) ADDRESSEE: Genentech, Inc.
      (B) STREET: 460 Point San Bruno Blvd
      (C) CITY: South San Francisco
      (D) STATE: California
      (E) COUNTRY: USA
      (F) ZIP: 94080
   (v) COMPUTER READABLE FORM:
      (A) MEDIUM TYPE: 3.5 inch, 1.44 Mb floppy disk
      (B) COMPUTER: IBM PC compatible
      (C) OPERATING SYSTEM: PC-DOS/MS-DOS
      (D) SOFTWARE: WinPatin (Genentech)
   (vi) CURRENT APPLICATION DATA:
      (A) APPLICATION NUMBER:
      (B) FILING DATE:
      (C) CLASSIFICATION:
   (viii) ATTORNEY/AGENT INFORMATION:
      (A) NAME: Dreger, Ginger R.
      (B) REGISTRATION NUMBER: 33,055
      (C) REFERENCE/DOCKET NUMBER: P1010PCT
   (ix) TELECOMMUNICATION INFORMATION:
      (A) TELEPHONE: 415/225-3216
      (B) TELEFAX: 415/952-9881
      (C) TELEX: 910/371-7168
(2) INFORMATION FOR SEQ ID NO:1:
   (i) SEQUENCE CHARACTERISTICS:
      (A) LENGTH: 1529 base pairs
      (B) TYPE: Nucleic Acid
      (C) STRANDEDNESS: Single
      (D) TOPOLOGY: Linear
   (xi) SEQUENCE DESCRIPTION: SEQ ID NO:1:
   CTCAGAGCGG GTCGCAGCAT GAGTCGCCAT ACGGACTTGG TGAGGAGCTT 50
   CTTGGAGCAG CTGGAGGCCC GGGACTACCG GGAGGGGGCA ATCTTCGTTC 100
   GTGAGTTCAG CGACATTAAG GCCCGCTCAG TGGCCTGGAA GTCTGAAGGT 150
   GTGTGTTCCA CTAAAGCCGG CAGTCGGCTT GGGAACACGA ACAAGAACCG 200
   CTACAAAGAT GTGGTAGCAT ATGATGAGAC AAGAGTCATC CTTTCCCTGC 250
   TCCAAGAGGA GGGACATGGA AATTACATCA ATGCCAACTT CATCCGGGGC 300
   ATAGATGGAA GCCAGGCCTA CATTGCGACG CAAGGACCCC TGCCTCACAC 350
   ACTGTTGGAC TTCTGGCGCC TGGTTTGGGA GTTTGGGGTC AAGGTAATCC 400
   TGATGGCCTG TCAAGAGACA GAAAATGGAC GGAGGAAGTG TGAACGCTAT 450
   TGGGCCCGGG AGCAGGAGCC TCTAAAGGCT GGGCCTTTCT GCATCACCCT 500
   GACAAAGGAG ACAACACTGA ATGCAGACAT CACTCTCAGG ACCCTCCAGG 550
   TTACATTCCA GAAGGAATTC CGCTCTGTGC ACCAACTACA GTATATGTCC 600
   TGGCCAGACC ACGGGGTTCC CAGCAGTTCT GATCACATTC TCACCATGGT 650
   GGAGGAGGCC CGCTGCCTCC AAGGGCTTGG ACCTGGACCC CTCTGTGTCC 700
   ACTGCAGTGC TGGCTGCGGA CGAACAGGTG TCCTGTGCGC TGTTGACTAT 750
   GTGAGGCAGT TGCTGCTGAC CCAGACAATC CCTCCCAACT TCAGTCTCTT 800
   CCAAGTGGTC CTGGAGATGC GGAAACAGCG GCCTGCAGCA GTGCAGACAG 850
   AGGAGCAGTA CAGGTTCCTG TACCACACAG TGGCTCAGCT ATTCTCCCGC 900
   ACTCTCCAGG ACACCAGCCC CCAATACCAG AACCTCAAGG AGAACTGCGC 950
   TCCAATCTGC AAGGAAGCTT TCTCCCTCAG GACCTCCTCA GCCCTGCCTG 1000
   CCACATCCCG GCCACCAGGA GGGGTTCTCA GGAGCATCTC GGTGCCTGCG 1050
   CCCCCGACCC TCCCCATGGC TGACACTTAC GCTGTGGTGC AGAAGCGTGG 1100
   CGCTTCGGCG GGCACAGGGC CGGGGCCGCG GGCGCCCACC AGCACGGACA 1150
   CCCCGATTTA CAGCCAGGTG GCTCCACGTG CCCAGCGACC GGTGGCACAC 1200
   ACGGAGGACG CACAGGGGAC AACGGCACTG CGCCGAGTTC CTGCGGACCA 1250
   AAACTCTTCC GGGCCTGATG CCTACGAAGA AGTAACAGAT GGAGCACAGA 1300
   CTGGAGGGCT AGGCTTCAAC TTGCGCATCG GAAGGCCCAA AGGGCCCCGG 1350
   GATCCTCCAG CAGAGTGGAC ACGGGTGTAA CGAGTGCTGT GCCAGTTATA 1400
   GCCTGCCACT CGGTGGTGGC TGGACTCCTG GAACCACCAT ACTGCTGTGC 1450
   AGTGTGTTAT GTATGAGTGG GACTTGTGGG CCTGATTCAA AATAAAAGTT 1500
   TCTCAGGGCG GAAAAAAAAA AAAAAAAAA 1529
(2) INFORMATION FOR SEQ ID NO:2:
   (i) SEQUENCE CHARACTERISTICS:
      (A) LENGTH: 453 amino acids
      (B) TYPE: Amino Acid
      (D) TOPOLOGY: Linear
   (xi) SEQUENCE DESCRIPTION: SEQ ID NO:2:
   Met Ser Arg His Thr Asp Leu Val Arg Ser Phe Leu Glu Gln Leu
    1               5                  10                  15
   Glu Ala Arg Asp Tyr Arg Glu Gly Ala Ile Phe Val Arg Glu Phe
                   20                  25                  30
   Ser Asp Ile Lys Ala Arg Ser Val Ala Trp Lys Ser Glu Gly Val
                   35                  40                  45
   Cys Ser Thr Lys Ala Gly Ser Arg Leu Gly Asn Thr Asn Lys Asn
                   50                  55                  60
   Arg Tyr Lys Asp Val Val Ala Tyr Asp Glu Thr Arg Val Ile Leu
                   65                  70                  75
   Ser Leu Leu Gln Glu Glu Gly His Gly Asn Tyr Ile Asn Ala Asn
   80 85 90
   Phe Ile Arg Gly Ile Asp Gly Ser Gln Ala Tyr Ile Ala Thr Gln
   95 100 105
   Gly Pro Leu Pro His Thr Leu Leu Asp Phe Trp Arg Leu Val Trp
   110 115 120
   Glu Phe Gly Val Lys Val Ile Leu Met Ala Cys Gln Glu Thr Glu
   125 130 135
   Asn Gly Arg Arg Lys Cys Glu Arg Tyr Trp Ala Arg Glu Gln Glu
   140 145 150
   Pro Leu Lys Ala Gly Pro Phe Cys Ile Thr Leu Thr Lys Glu Thr
   155 160 165
   Thr Leu Asn Ala Asp Ile Thr Leu Arg Thr Leu Gln Val Thr Phe
   170 175 180
   Gln Lys Glu Phe Arg Ser Val His Gln Leu Gln Tyr Met Ser Trp
   185 190 195
   Pro Asp His Gly Val Pro Ser Ser Ser Asp His Ile Leu Thr Met
   200 205 210
   Val Glu Glu Ala Arg Cys Leu Gln Gly Leu Gly Pro Gly Pro Leu
   215 220 225
   Cys Val His Cys Ser Ala Gly Cys Gly Arg Thr Gly Val Leu Cys
   230 235 240
   Ala Val Asp Tyr Val Arg Gln Leu Leu Leu Thr Gln Thr Ile Pro
   245 250 255
   Pro Asn Phe Ser Leu Phe Gln Val Val Leu Glu Met Arg Lys Gln
   260 265 270
   Arg Pro Ala Ala Val Gln Thr Glu Glu Gln Tyr Arg Phe Leu Tyr
   275 280 285
   His Thr Val Ala Gln Leu Phe Ser Arg Thr Leu Gln Asp Thr Ser
   290 295 300
   Pro Gln Tyr Gln Asn Leu Lys Glu Asn Cys Ala Pro Ile Cys Lys
   305 310 315
   Glu Ala Phe Ser Leu Arg Thr Ser Ser Ala Leu Pro Ala Thr Ser
   320 325 330
   Arg Pro Pro Gly Gly Val Leu Arg Ser Ile Ser Val Pro Ala Pro
   335 340 345
   Pro Thr Leu Pro Met Ala Asp Thr Tyr Ala Val Val Gln Lys Arg
   350 355 360
   Gly Ala Ser Ala Gly Thr Gly Pro Gly Pro Arg Ala Pro Thr Ser
   365 370 375
   Thr Asp Thr Pro Ile Tyr Ser Gln Val Ala Pro Arg Ala Gln Arg
   380 385 390
   Pro Val Ala His Thr Glu Asp Ala Gln Gly Thr Thr Ala Leu Arg
   395 400 405
   Arg Val Pro Ala Asp Gln Asn Ser Ser Gly Pro Asp Ala Tyr Glu
                   410                 415                 420
   Glu Val Thr Asp Gly Ala Gln Thr Gly Gly Leu Gly Phe Asn Leu
                   425                 430                 435
   Arg Ile Gly Arg Pro Lys Gly Pro Arg Asp Pro Pro Ala Glu Trp
                   440                 445                 450
   Thr Arg Val
           453
(2) INFORMATION FOR SEQ ID NO:3:
   (i) SEQUENCE CHARACTERISTICS:
      (A) LENGTH: 39 base pairs
      (B) TYPE: Nucleic Acid
      (C) STRANDEDNESS: Single
      (D) TOPOLOGY: Linear
   (xi) SEQUENCE DESCRIPTION: SEQ ID NO:3:
   CACGGTCGAC GGTGAGGAGC TTCTTTGAGC AGCTGGAGG 39
(2) INFORMATION FOR SEQ ID NO:4:
   (i) SEQUENCE CHARACTERISTICS:
      (A) LENGTH: 42 base pairs
      (B) TYPE: Nucleic Acid
      (C) STRANDEDNESS: Single
      (D) TOPOLOGY: Linear
   (xi) SEQUENCE DESCRIPTION: SEQ ID NO:4:
   GTTGCGGCCG CGATTGGAGC GCAGTTCTCC TTGAGGTTCT GG 42
(2) INFORMATION FOR SEQ ID NO:5:
   (i) SEQUENCE CHARACTERISTICS:
      (A) LENGTH: 36 base pairs
      (B) TYPE: Nucleic Acid
      (C) STRANDEDNESS: Single
      (D) TOPOLOGY: Linear
   (xi) SEQUENCE DESCRIPTION: SEQ ID NO:5:
   CCTGGAGGGT CCTGAGAGTG ATGTCTGCAT TCAGTG 36
(2) INFORMATION FOR SEQ ID NO:6:
   (i) SEQUENCE CHARACTERISTICS:
      (A) LENGTH: 34 base pairs
      (B) TYPE: Nucleic Acid
      (C) STRANDEDNESS: Single
      (D) TOPOLOGY: Linear
   (xi) SEQUENCE DESCRIPTION: SEQ ID NO:6:
   CCTCTTGGAG CAGGGAAAGG ATGACTCTTG TCTC 34
(2) INFORMATION FOR SEQ ID NO:7:
   (i) SEQUENCE CHARACTERISTICS:
      (A) LENGTH: 30 base pairs
      (B) TYPE: Nucleic Acid
      (C) STRANDEDNESS: Single
      (D) TOPOLOGY: Linear
   (xi) SEQUENCE DESCRIPTION: SEQ ID NO:7:
   CAGCTGCTCC AAGAAGCTCC TCACCAAGTC 30
(2) INFORMATION FOR SEQ ID NO:8:
   (i) SEQUENCE CHARACTERISTICS:
      (A) LENGTH: 31 base pairs
      (B) TYPE: Nucleic Acid
      (C) STRANDEDNESS: Single
      (D) TOPOLOGY: Linear
   (xi) SEQUENCE DESCRIPTION: SEQ ID NO:8:
   GGTAGAGGTG GGCAGGGTGA AGTGTTCTCG C 31
(2) INFORMATION FOR SEQ ID NO:9:
   (i) SEQUENCE CHARACTERISTICS:
      (A) LENGTH: 36 base pairs
      (B) TYPE: Nucleic Acid
      (C) STRANDEDNESS: Single
      (D) TOPOLOGY: Linear
   (xi) SEQUENCE DESCRIPTION: SEQ ID NO:9:
   CACTGAATGC AGACATCACT CTCAGGACCC TCCAGG 36
(2) INFORMATION FOR SEQ ID NO:10:
   (i) SEQUENCE CHARACTERISTICS:
      (A) LENGTH: 34 base pairs
      (B) TYPE: Nucleic Acid
      (C) STRANDEDNESS: Single
      (D) TOPOLOGY: Linear
   (xi) SEQUENCE DESCRIPTION: SEQ ID NO:10:
   GAGACAAGAG TCATCCTTTC CCTGCTCCAA GAGG 34
(2) INFORMATION FOR SEQ ID NO:11:
   (i) SEQUENCE CHARACTERISTICS:
      (A) LENGTH: 25 base pairs
      (B) TYPE: Nucleic Acid
      (C) STRANDEDNESS: Single
      (D) TOPOLOGY: Linear
   (xi) SEQUENCE DESCRIPTION: SEQ ID NO:11:
   GAATGGTAAC CTGGAGGGTC CTGAG 25
(2) INFORMATION FOR SEQ ID NO:12:
   (i) SEQUENCE CHARACTERISTICS:
      (A) LENGTH: 18 base pairs
      (B) TYPE: Nucleic Acid
      (C) STRANDEDNESS: Single
      (D) TOPOLOGY: Linear
   (xi) SEQUENCE DESCRIPTION: SEQ ID NO:12:
   GAGAAGGTCG TGTTCGAG 18
(2) INFORMATION FOR SEQ ID NO:13:
   (i) SEQUENCE CHARACTERISTICS:
      (A) LENGTH: 18 base pairs
      (B) TYPE: Nucleic Acid
      (C) STRANDEDNESS: Single
      (D) TOPOLOGY: Linear
   (xi) SEQUENCE DESCRIPTION: SEQ ID NO:13:
   GTGTACTTCC TGTGCCTG 18
(2) INFORMATION FOR SEQ ID NO:14:
   (i) SEQUENCE CHARACTERISTICS:
      (A) LENGTH: 18 base pairs
      (B) TYPE: Nucleic Acid
      (C) STRANDEDNESS: Single
      (D) TOPOLOGY: Linear
   (xi) SEQUENCE DESCRIPTION: SEQ ID NO:14:
   ANTTNTGGNG ATGNTTGG 18
(2) INFORMATION FOR SEQ ID NO:15:
   (i) SEQUENCE CHARACTERISTICS:
      (A) LENGTH: 15 base pairs
      (B) TYPE: Nucleic Acid
      (C) STRANDEDNESS: Single
      (D) TOPOLOGY: Linear
   (xi) SEQUENCE DESCRIPTION: SEQ ID NO:15:
   GGACNNNNTC GGCCA 15
(2) INFORMATION FOR SEQ ID NO:16:
   (i) SEQUENCE CHARACTERISTICS:
      (A) LENGTH: 466 base pairs
      (B) TYPE: Nucleic Acid
      (C) STRANDEDNESS: Single
      (D) TOPOLOGY: Linear
   (xi) SEQUENCE DESCRIPTION: SEQ ID NO:16:
   GCGCGGGGCG GCCGGGAGGG GGCAGTCCTC GCCGGCGAGT TCAGCGACAT 50
   CCAGGCCTGC TCGGCCGCCT GGAAGGCTGA CGGCGTGTGC TCCACCGTGG 100
   CCGGCAGTCG GCCAGAGAAC GTGAGGAAGA ACCGCTACAA AGACGTGCTG 150
   CCTTATGATC AGACGCGAGT AATCCTCTCC CTGCTCCAGG AAGAGGGACA 200
   CAGCGACTAC ATTAATGGCA ACTTCATCCG GGGCGTGGAT GGAAGCCTGG 250
   CCTACATTGC CACGCAAGGA CCCTTGCCTC ACACCCTGCT AGACTTCTGG 300
   AGACTGGTCT GGGAGTTTGG GGTCAAGGTG ATCCTGATGG CCTGTCGAGA 350
   GATAGAGAAT GGGCGGAAAA GGTGTGAGCG GTACTGGGCC CAGGAGCAGG 400
   AGCCACTGCA GACTGGGCTT TTCTGCATCA CTCTGATAAA GGAGAAGTGG 450
   CTGAATGAGG ACATCA 466
(2) INFORMATION FOR SEQ ID NO:17:
   (i) SEQUENCE CHARACTERISTICS:
      (A) LENGTH: 155 amino acids
      (B) TYPE: Amino Acid
      (D) TOPOLOGY: Linear
   (xi) SEQUENCE DESCRIPTION: SEQ ID NO:17:
   Ala Arg Gly Gly Arg Glu Gly Ala Val Leu Ala Gly Glu Phe Ser
    1               5                  10                  15
   Asp Ile Gln Ala Cys Ser Ala Ala Trp Lys Ala Asp Gly Val Cys
                   20                  25                  30
   Ser Thr Val Ala Gly Ser Arg Pro Glu Asn Val Arg Lys Asn Arg
                   35                  40                  45
   Tyr Lys Asp Val Leu Pro Tyr Asp Gln Thr Arg Val Ile Leu Ser
                   50                  55                  60
   Leu Leu Gln Glu Glu Gly His Ser Asp Tyr Ile Asn Gly Asn Phe
                   65                  70                  75
   Ile Arg Gly Val Asp Gly Ser Leu Ala Tyr Ile Ala Thr Gln Gly
                   80                  85                  90
   Pro Leu Pro His Thr Leu Leu Asp Phe Trp Arg Leu Val Trp Glu
                   95                  100                 105
   Phe Gly Val Lys Val Ile Leu Met Ala Cys Arg Glu Ile Glu Asn
                   110                 115                 120
   Gly Arg Lys Arg Cys Glu Arg Tyr Trp Ala Gln Glu Gln Glu Pro
                   125                 130                 135
   Leu Gln Thr Gly Leu Phe Cys Ile Thr Leu Ile Lys Glu Lys Trp
                   140                 145                 150
   Leu Asn Glu Asp Ile
                   155
(2) INFORMATION FOR SEQ ID NO:18:
   (i) SEQUENCE CHARACTERISTICS:
      (A) LENGTH: 278 amino acids
      (B) TYPE: Amino Acid
      (D) TOPOLOGY: Linear
   (xi) SEQUENCE DESCRIPTION: SEQ ID NO:18:
   Phe Ala Ser Glu Phe Leu Lys Leu Lys Arg Gln Ser Thr Lys Tyr
    1               5                  10                  15
   Lys Ala Asp Lys Ile Tyr Pro Thr Thr Val Ala Gln Arg Pro Lys
                   20                  25                  30
   Asn Ile Lys Lys Asn Arg Tyr Lys Asp Ile Leu Pro Tyr Asp His
                   35                  40                  45
   Ser Leu Val Glu Leu Ser Leu Leu Thr Ser Asp Glu Asp Ser Ser
                   50                  55                  60
   Tyr Ile Asn Ala Ser Phe Ile Lys Gly Val Tyr Gly Pro Lys Ala
                   65                  70                  75
   Tyr Ile Ala Thr Gln Gly Pro Leu Ser Thr Thr Leu Leu Asp Phe
                   80                  85                  90
   Trp Arg Met Ile Trp Glu Tyr Arg Ile Leu Val Ile Val Met Ala
                   95                  100                 105
   Cys Met Glu Phe Glu Met Gly Lys Lys Lys Cys Glu Arg Tyr Trp
                   110                 115                 120
   Ala Glu Pro Gly Glu Thr Gln Leu Gln Phe Gly Pro Phe Ser Ile
                   125                 130                 135
   Ser Cys Glu Ala Glu Lys Lys Lys Ser Asp Tyr Lys Ile Arg Thr
                   140                 145                 150
   Leu Lys Ala Lys Phe Asn Asn Glu Thr Arg Ile Ile Tyr Gln Phe
                   155                 160                 165
   His Tyr Lys Asn Trp Pro Asp His Asp Val Pro Ser Ser Ile Asp
                   170                 175                 180
   Pro Ile Leu Gln Leu Ile Trp Asp Met Arg Cys Tyr Gln Glu Asp
                   185                 190                 195
   Asp Cys Val Pro Ile Cys Ile His Cys Ser Ala Gly Cys Gly Arg
                   200                 205                 210
   Thr Gly Val Ile Cys Ala Val Asp Tyr Thr Trp Met Leu Leu Lys
                   215                 220                 225
   Asp Gly Ile Ile Pro Lys Asn Phe Ser Val Phe Asn Leu Ile Gln
                   230                 235                 240
   Glu Met Arg Thr Gln Arg Pro Ser Leu Val Gln Thr Gln Glu Gln
                   245                 250                 255
   Tyr Glu Leu Val Tyr Ser Ala Val Leu Glu Leu Phe Lys Arg His
                   260                 265                 270
   Met Asp Val Ile Ser Asp Asn His
                   275         278
(2) INFORMATION FOR SEQ ID NO:19:
   (i) SEQUENCE CHARACTERISTICS:
      (A) LENGTH: 272 amino acids
      (B) TYPE: Amino Acid
      (D) TOPOLOGY: Linear
   (xi) SEQUENCE DESCRIPTION: SEQ ID NO:19:
   Phe Ala Arg Asp Phe Met Arg Leu Arg Arg Leu Ser Thr Lys Tyr
    1               5                  10                  15
   Arg Thr Glu Lys Ile Tyr Pro Thr Ala Thr Gly Glu Lys Glu Glu
                   20                  25                  30
   Asn Val Lys Lys Asn Arg Tyr Lys Asp Ile Leu Pro Phe Asp His
                   35                  40                  45
   Ser Arg Val Lys Leu Thr Leu Lys Thr Pro Ser Gln Asp Ser Asp
                   50                  55                  60
   Tyr Ile Asn Ala Asn Phe Ile Lys Gly Val Tyr Gly Pro Lys Ala
                   65                  70                  75
   Tyr Val Ala Thr Gln Gly Pro Leu Ala Asn Thr Val Ile Asp Phe
                   80                  85                  90
   Trp Arg Met Val Trp Glu Tyr Asn Val Val Ile Ile Val Met Ala
                   95                  100                 105
   Cys Arg Glu Phe Glu Met Gly Arg Lys Lys Cys Glu Arg Tyr Trp
                   110                 115                 120
   Pro Leu Tyr Gly Glu Asp Pro Ile Thr Phe Ala Pro Phe Lys Ile
                   125                 130                 135
   Ser Cys Glu Asp Glu Gln Ala Arg Thr Asp Tyr Phe Ile Arg Thr
                   140                 145                 150
   Leu Leu Leu Glu Phe Gln Asn Glu Ser Arg Arg Leu Tyr Gln Phe
                   155                 160                 165
   His Tyr Val Asn Trp Pro Asp His Asp Val Pro Ser Ser Phe Asp
                   170                 175                 180
   Ser Ile Leu Asp Met Ile Ser Leu Met Arg Lys Tyr Gln Glu His
                   185                 190                 195
   Glu Asp Val Pro Ile Cys Ile His Cys Ser Ala Gly Cys Gly Arg
                   200                 205                 210
   Thr Gly Ala Ile Cys Ala Ile Asp Tyr Thr Trp Asn Leu Leu Lys
                   215                 220                 225
   Ala Gly Lys Ile Pro Glu Glu Phe Asn Val Phe Asn Leu Ile Gln
                   230                 235                 240
   Glu Met Arg Thr Gln Arg His Ser Ala Val Gln Thr Lys Glu Gln
                   245                 250                 255
   Tyr Glu Leu Val His Arg Ala Ile Ala Gln Leu Phe Glu Lys Gln
                   260                 265                 270
   Leu Gln
       272
(2) INFORMATION FOR SEQ ID NO:20:
   (i) SEQUENCE CHARACTERISTICS:
      (A) LENGTH: 33 base pairs
      (B) TYPE: Nucleic Acid
      (C) STRANDEDNESS: Single
      (D) TOPOLOGY: Linear
   (xi) SEQUENCE DESCRIPTION: SEQ ID NO:20:
   ACTTGGTGAG GAGCTTCTTG GAGCAGCTGG AGG 33
(2) INFORMATION FOR SEQ ID NO:21:
   (i) SEQUENCE CHARACTERISTICS:
      (A) LENGTH: 24 base pairs
      (B) TYPE: Nucleic Acid
      (C) STRANDEDNESS: Single
      (D) TOPOLOGY: Linear
   (xi) SEQUENCE DESCRIPTION: SEQ ID NO:21:
   GGAATGTAAC CTGGAGGGTC CTGA 24
(2) INFORMATION FOR SEQ ID NO:22:
   (i) SEQUENCE CHARACTERISTICS:
      (A) LENGTH: 19 amino acids
      (B) TYPE: Amino Acid
      (D) TOPOLOGY: Linear
   (xi) SEQUENCE DESCRIPTION: SEQ ID NO:22:
   Gly Phe Gly Asn Arg Phe Ser Lys Pro Lys Gly Pro Arg Asn Pro
    1               5                   10                  15
   Pro Ser Ala Trp
               19
(2) INFORMATION FOR SEQ ID NO:23:
   (i) SEQUENCE CHARACTERISTICS:
      (A) LENGTH: 21 amino acids
      (B) TYPE: Amino Acid
      (D) TOPOLOGY: Linear
   (xi) SEQUENCE DESCRIPTION: SEQ ID NO:23:
   Ile Gly Phe Gly Asn Arg Cys Gly Lys Pro Lys Gly Pro Arg Asp
     1             5                  10                  15
   Pro Pro Ser Glu Trp Thr
                  20  21

## Description

### Field of the Invention

The present invention concerns novel protein tyrosine phosphatases. More particularly, the invention concerns non-receptor protein tyrosine phosphatases of hematopoietic stem cells (PTP HSC's).

### Background of the Invention

The ability of the hematopoietic stem cell to function as a source of committed progenitors throughout the lifetime of the organism is, at present, a poorly understood phenomenon. The major characteristic of the hematopoietic stem cell is its ability to self renew in the absence of differentiation (Morrison *et al*., Ann. Rev. Cell Dev. Biol., 11, 35-71 [1995]). This self renewal phenomenon is especially remarkable in light of the fact that the hematopoietic stroma, which is in close physical contact with the stem cell, is known to be a source that is rich in factors which mediate the growth and differentiation of hematopoietic progenitors (Deryugina and Muller-Sieberg, Crit. Rev. in Immunol. 13(2), 115-150 [1993]). For example, a recent PCR analysis of hematopoietically active endothelial cell stromal lines derived from the murine yolk sac revealed that these cells produced a plethora of growth and differentiation factors including stem cell factor, FLT 3 ligand, M-CSF, LIF and IL-6 (Fennie *et al*., Blood 86(12), 4454-4467 [1995]). Such growth factors, in addition to many others, are known to induce the expansion and differentiation of stem cells, and these endothelial cell lines induced a rapid expansion and differentiation of embryonic hematopoietic stem cells along the myeloid pathway, although very early progenitor cells are also amplified by these stromal cells (C. Fennie and L. Lasky-unpublished data). It has also been shown that incubation of highly purified stem cell populations in the presence of various purified hematopoietic growth factors induces differentiation with subsequent loss of the cells' ability to competitively repopulate the hematopoietic compartment oflethally irradiated animals, consistent with the induction of terminal differentiation (Peters *et al*., Blood 87(1): 30-37 [1996]). Thus, the stem cell, whether in an embryonic or adult lt stromal environment, must maintain an undifferentiated state in spite of the fact that it is being exposed to a variety such maturation factors (Deryugina and Muller-Sieberg, *supra).*

Although the hematopoietic growth factors are very diverse both structurally and functionally, they are all believed to play role in mediating protein phosphorylation (Paulson and Bernstein, Semin lmmunol. 7(4), 267-77 [1995]). This protein modification can occur via direct means, such as in the cases of the stem cell factor and FLT-3 receptors, both of which have intrinsic tyrosine kinase activity, or via indirect means, as is the case of the hematopoietic/cytokine growth factor receptors for, for example, IL-3, EPO and TPO. In the case of the hematopoietic/cytokine growth factor receptors, tyrosine phosphorylation is indirectly accomplished by the activation of the JAK kinases, which occurs after growth factor mediated receptor dimerization (Ihle *et al*., Annu. Rev. Immunol. 13, 369-398 [1995]). In both cases, diverse complex pathways of protein phosphorylation are stimulated upon receptor binding. The intrinsic tyrosine kinase receptors mediate their signals via an elaborate series of tyrosine phosphorylation events which ultimately activate the RAS signaling pathway (Fantl *et al*., Ann. Rev. Biochem. 62, 453-481 [1993]). This pathway eventually leads to the activation of the serine/threonine specific MAP kinase pathway which results in transcriptional activation. In contrast to this intricate pathway, hematopoietic growth factor-induced receptor dimerization mediates more direct activation events. Thus, the stimulation of the JAK kinases by receptor binding leads to the tyrosine phosphorylation and subsequent dimerization of various STAT proteins. These activated STAT proteins than migrate to the nucleus, bind to STAT responsive sites in the nuclear DNA and induce transcription of differentiation and growth specific genes. Thus, a major effect of the growth factors produced by the hematopoietic stroma is to mediate the activation of various cellular pathways by protein phosphorylation.

The regulation of protein tyrosine phosphorylation is accomplished by a balance between protein tyrosine kinases and protein tyrosine phosphatases (PTPs) (Walton and Dixon, Ann. Rev. Biochem. 62 , 101-120 [1993]; Sun and Tonks, Trends Biochem. Sci., 19(11), 480-485 [1994]). All PTPs contain a phosphatase domain including a subset of highly conserved amino acids, and a recent crystal structure analysis of PTP 1B complexed with a tyrosine phosphorylated peptide revealed that many of these conserved residues are involved with substrate recognition and tyrosine dephosphorylation (Jia *et al*., Science 268(5218), 1754-1758 [1995]). PTPs fall into two general categories: receptor type and non-receptor type. The receptor type PTPs have variously sized extracellular domains and, generally, two intracellular phosphatase domains Walton and Doxin, *supra*; Sun and Tonks, *supra.* The extracellular domains often contain a number of motifs that are generally utilized in cell adhesion including immunoglobulin domains and fibronectin-like regions . Many of these PTPₛ appear to function as homotypic and heterotypic sensors of the extracellular space, and they have been hypothesized to play roles in contact inhibition, cell guidance and other intercellular functions (Brady-Kalnay and Tonks, Curr. Opin. Cell. Biol. 7(5), 65-657 [1995]). The non-receptor PTPs are generally intracellular enzymes. They have various cellular localizations, depending upon the types of domains they contain, and some of the enzymes contain SH2 motifs which allow them to interact intimately with phosphotyrosine residues. While many of the non-receptor PTPs are in various cytoplasmic locations, a small number of these enzymes are found in the nucleus (Flores *et al*., Mol. Cell. Biol. 14(7), 4938-46 [1994]). Many non-receptor PTPs appear to function as both activators as well as inhibitors of diverse tyrosine phosphorylated proteins. A subset appear to play important rotes in hematopoiesis. For example, the motheaten mouse, which has a phenotype of lethal myeloid amplification and inflammation, has been found to have a mutation in the PTP I C gene (Schulz *et al*., Cell 73(7), 1445-54 [1993]); (McCulloch and Siminovitch, Adv. Exp. Med. Biol. 365, 145-54 [1994]). In addition, the level of tyrosine phosphorylation of the EPO receptor, as well as the level of receptor activation, appears to be in part controlled by the PTP 1C enzyme as well (Klingmuller *et al*., Cell 80(5), 729-38 [1995]). However, while these examples, as well as others, highlight the potential importance of the PTPs, very little is known regarding the physiological importance of these enzymes.

Yi et al (1991) Blood 78(9), 2222-2228 describes a non-PTP termed PTPTY42. WO 01/13989 describes methods and compositions for treating malignancies, using a DNA sequence encoding a PTPase.

### Summary of the Invention

We have hypothesized that one mechanism by which the undifferentiated state of the stem cell might be maintained is by the dephosphorylation of tyrosine phosphorylated proteins by PTPs. In order to examine this possibility, we have analyzed a large number of PTPs from a very primitive embryonic hematopoietic cell population using consensus PCR. From this population we have cloned a novel intracellular PTP which has many of the characteristics, including down-regulation of the transcript as the hematopoietic stem cells differentiate, which might be expected from a PTP involved with the control of differentiation signals such as those induced by hematopoetic growth factors. We have designated this novel PTP as the "PTP of hematopoietic stem cells", which will be referred to hereafter as "PTP HSC."

Accordingly, the present invention concerns an isolated non-receptor protein tyrosine phosphatase of hematopoietic stem cells (PTP HSC) as defined in claim 1 or claim 6. The PTP HSC
(1) is expressed predominantly in early hematopoietic stem/progenitor cells;
(2) predominantly lacks expression in adult tissues;
(3) comprises an N-terminal tyrosine phosphatase domain, followed by a region rich in serine, threonine, and proline, and a carboxy terminal region of about 15 to 25 amino acids rich in basic amino acid residues; and
(4) is capable of tyrosine dephosphorylation in hematopoietic stem cells or progenitor cells.

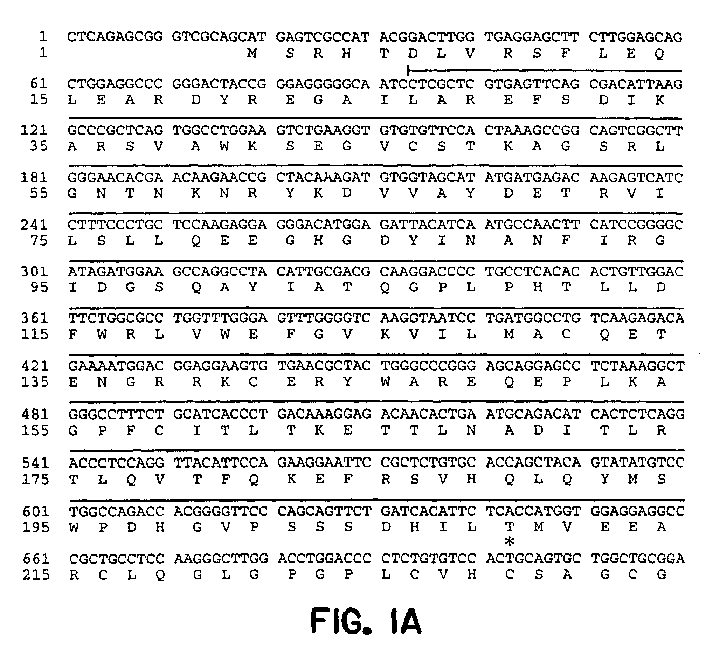

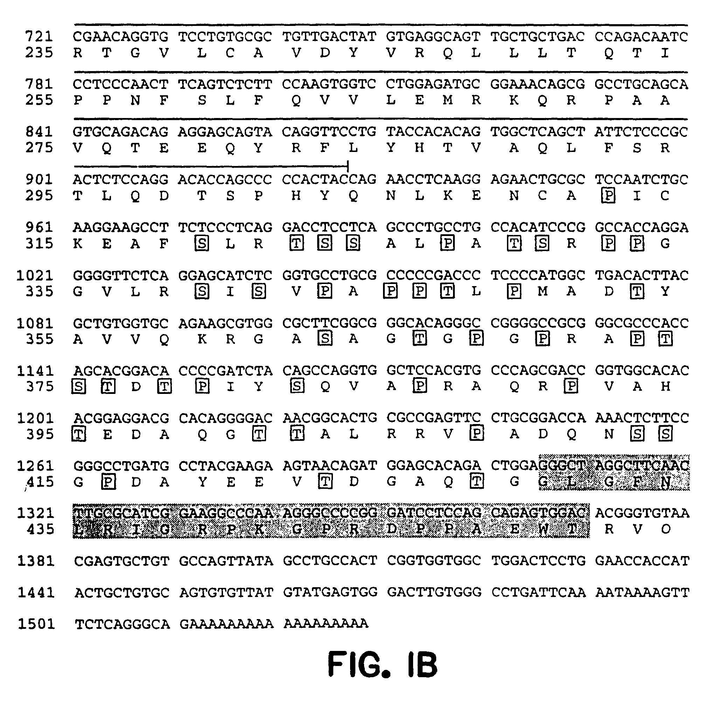

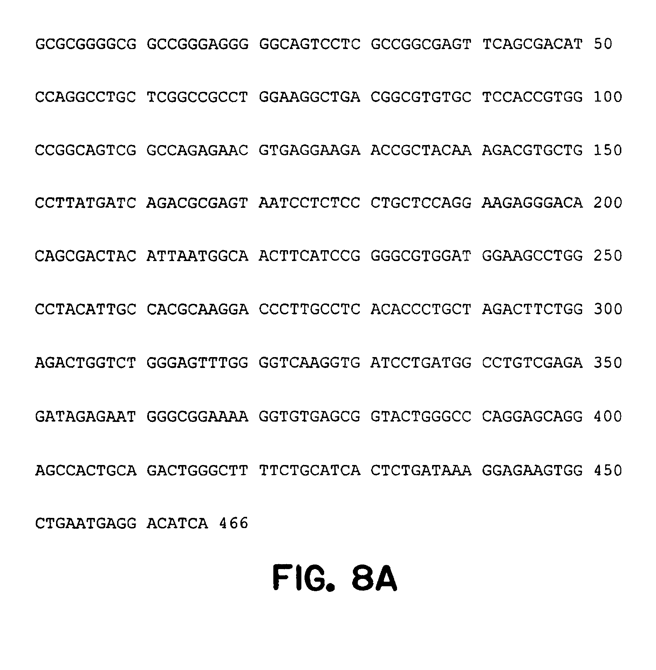

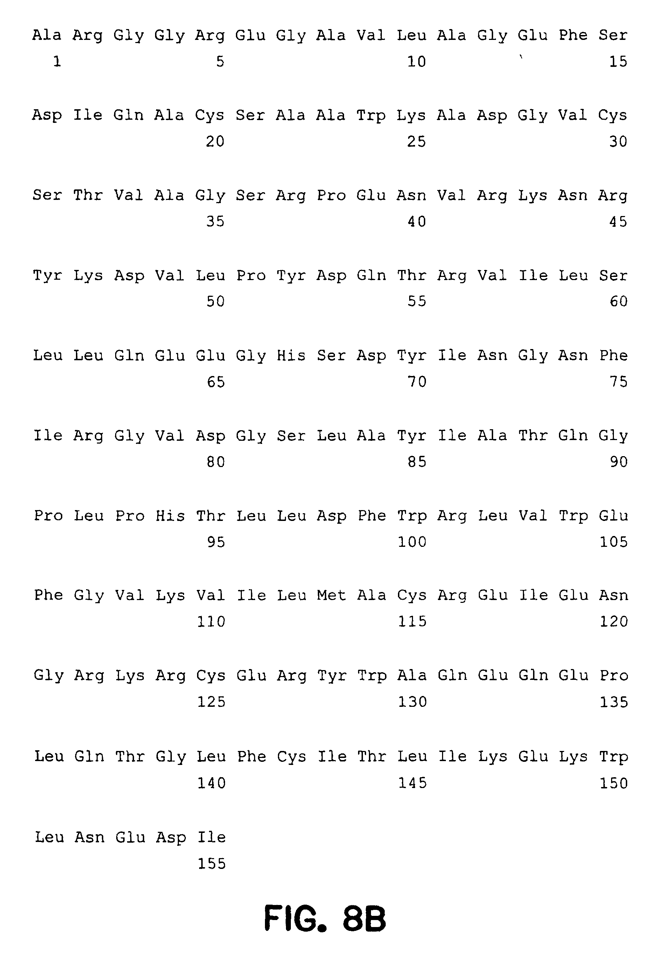

This novel PTP preferably downregulates STAT activation. A preferred group of the PTP HSC proteins of the present invention includes a protein comprising the amino acid sequence shown in Figure 1 (SEQ. ID. NO:2); a protein comprising the amino acid sequence shown in Figure 8 (SEQ. ID. NO: 17), a further mammalian homologue of either protein, as defined in claim 6; and derivatives of the foregoing proteins retaining the ability of tyrosine dephosphorylation in hematopoietic stem cells or progenitor cells.

The PTP HSCs, including derivatives (e.g. amino acid sequence variants) of the native proteins, preferably have an active N-terminal tyrosine phosphatase domain, retaining a serine residue at a position corresponding to amino acid position 37 in Figure 1, and retaining an active site cysteine residue at a position corresponding to amino acid position 229 in Figure 1, a region rich in serine, threonine, and proline, and a carboxy-terminal region showing at least about 80% sequence homology with the amino acid sequence between positions 430 and 451 in Figure 1. Most preferably, such derivatives have at least about 65% overall sequence homology with the amino acid sequence shown in Figure 1 or Figure 8 and retain the ability of tyrosine dephosphorylation in hematopoietic stem cells or progenitor cells.

In another aspect, the present invention concerns agonists and antagonists of PTP HSCs.

In yet another aspect, the invention concerns isolated nucleic acid molecules encoding the PTP HSCs herein.

In a further aspect, the invention concerns vectors comprising nucleic acid encoding the PTP HSCs herein, operably linked to control sequences recognized by a host cell transformed with the vector, and to cells transformed with such vectors.

In a still further aspect of the present invention, there are provided antibodies capable of specific binding to the PTP HSCs of this invention, and hybridoma cell lines producing such antibodies. The antibodies may be agonist antibodies, which stimulate the ability of the native PTP HSCs of the present invention to dephosphorylate tyrosines, or antagonist antibodies, which block this activity.

The present invention further concerns an assay for identifying an antagonist or an agonist of a PTP HSC of the present invention, which comprises contacting the phosphatase domain of the PTP HSC with a candidate antagonist or agonist, and monitoring the ability of the phosphatase domain to dephosphorylate tyrosine residues.

We provide an assay for identifying an antagonist or agonist of a PTP HSC of the present invention by cultivating a PTP HSC-expressing hematopoietic stem cell line or progenitor cell line in the presence of candidate antagonist or agonist, and monitoring the differentiation of the progenitor cells.

The invention further concerns a method for the differentiation of undifferentiated malignant hemopoietic (e.g. leukemia) cells in cell culture, comprising contacting said cells with an antagonist antibody of a PTP HSC of the present invention.

In an additional aspect, the invention concerns a method for the induction of hematopoietic stem cell differentiation in cell culture, comprising contacting said stem cells with an antagonist antibody of a PTP HSC of the present invention.

In another aspect, the invention concerns a method for expansion undifferentiated hematopoietic stems cells in cell culture, comprising cultivating stem cells in the presence of a PTP HSC of the present invention or an agonist antibody specifically binding a native PTP HSC.

In yet another aspect, the invention concerns the manufacture of a medicament, for example for the expansion of undifferentiated stem cells *in vivo* in one example, the medicament is for administering to a patient an agonist antibody of PTP HSC of the present invention ; and a stem cell growth factor.

### Brief Description of the Drawings

**Figure 1. DNA and deduced protein sequence of the murine PTP HSC cDNA.** Illustrated is the DNA sequence (SEQ. ID. NO: 1) and deduced protein sequence (SEQ. ID. NO: 2) of the murine PTP HSC cDNA. The overlined region is the phosphatase homologous domain. The asterisk denotes the active site cysteine residue. The P,S,T-rich region is illustrated by boxes around these residues. The shaded carboxy terminal region is homologous to a nuclear localization signal found on murine PTP PEP (Flores *et al*., Mol. Cell. Biol. 14(7), 4938-46 [1994]).

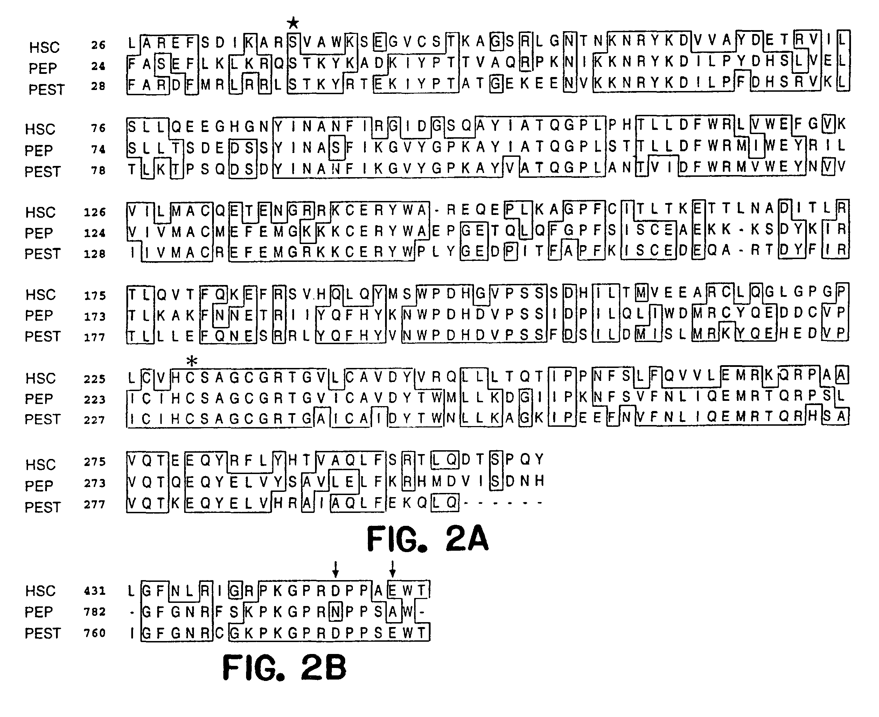

**Figure 2. Sequence homologies of murine PTP HSC, murine PTP PEP, and human PTP PEST. A.** The phosphatase domain homologies show that these three proteins are highly related to each other. A star over the residue (amino acid 37 ofPTP HSC) illustrates a conserved serine that is phosphorylated by protein kinases A and C and which appears to negatively regulate PTPase activity (Carton and Tonks, EMBO J. 13(16), 3763-71 [1994]). The amino acid sequence of positions 24 - 301 ofPTP PEP is shown in SEQ. ID. NO- 18; the amino acid sequence of positions 24 - 299 of PTP PEST is shown in SEQ. ID. NO: 19. B. A second highly homologous region is found at the carboxy terminus of these three proteins (SEQ. ID. NO: 22 showing amino acids 783 - 803 of PTP PEP; SEQ. ID. NO: 23 showing amino acids 761 - 781 of PTP PEST). This region has been shown to confer nuclear localization on PTP PEP. Interestingly PTP PEST is localized to the cytoplasm, and it has been hypothesized that this is due to the two negatively charged residues shown by the arrows. As can be seen, PTP HSC also contains these negatively charged residues, suggesting that it is also localized to the cytoplasm.

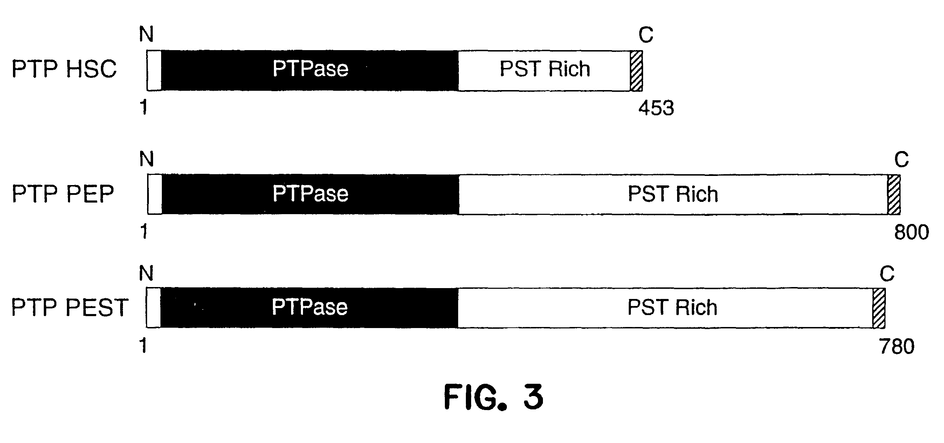

**Figure 3. The PTP PST family.** Illustrated are the three so far identified members of this family including the currently described novel PTP (PTP HSC). Shown are the amino terminal PTP domains (black), the P,S,T rich domains, and the carboxy terminal nuclear localization homology (shaded).

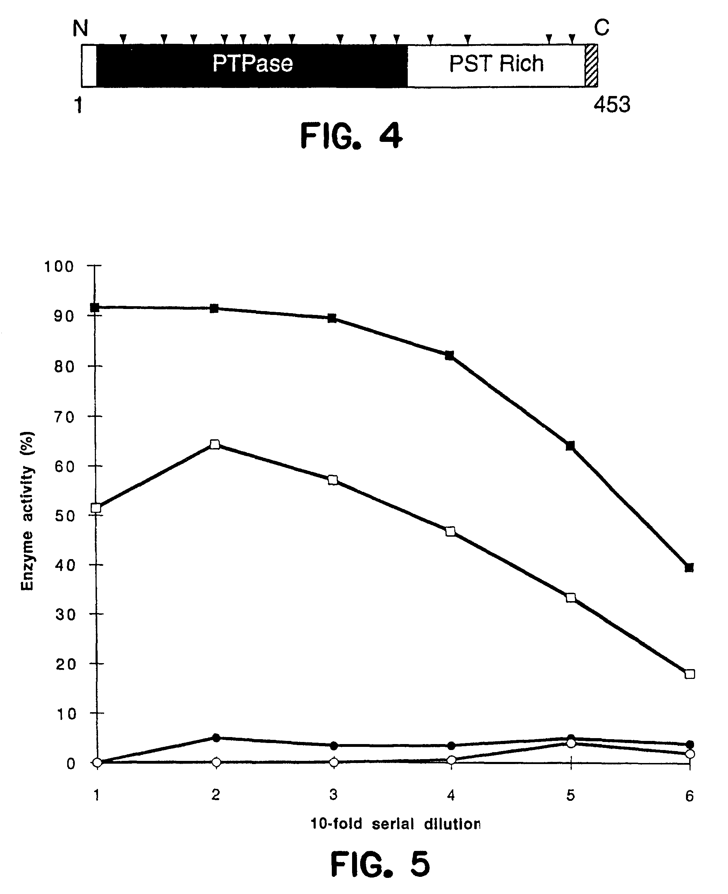

**Figure 4. Intron sites superimposed on the PTP HSC domain structure.** Analysis of the gene encoding PTP HSC revealed the location of 14 introns that are shown as triangles in this figure.
**Figure 5. In vitro tyrosine phosphatase activity of the PTP HSC.** Shown is the enzymatic activity obtained using isolated, bacterially produced GST-phosphatase domain of PTP HSC. Black squares, serial dilutions of GST-PTP HSC in the absence of orthovanadate; white squares, enzymatic activity of GST-PTP HSC in the presence of vanadate; closed circle, enzymatic activity of GST alone; open circles enzymatic activity with an inactive GST-PTP (J. Cheng and L. Lasky-unpublished data). The initial undiluted reaction contained 2 µg of each protein.

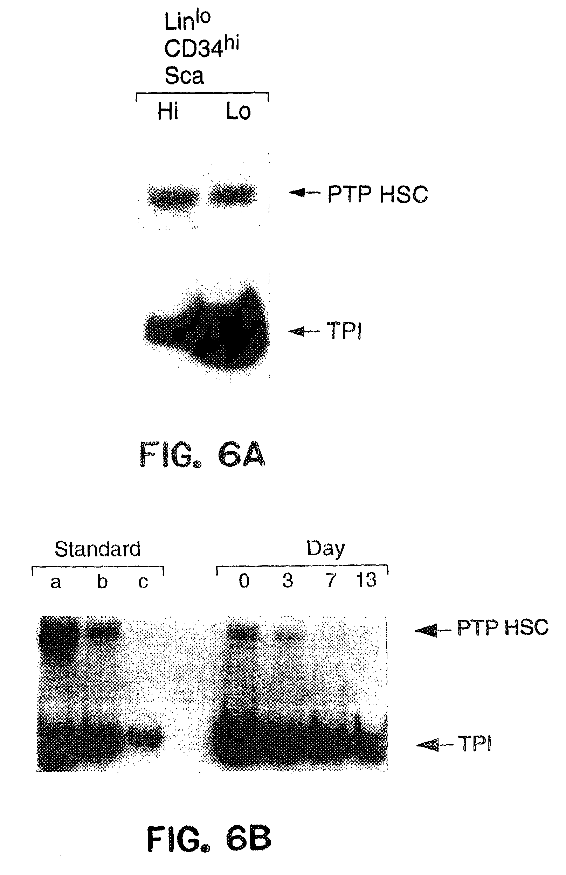

**Figure 6. PCR analysis of PTP HSC expression. A.** lin^{lo}CD34^{hi}sca^{hi} or lin^{lo}CD34^{hi}sca^{lo} hematopoietic progenitor cells were isolated from murine embryos at day ] of development. RNA was isolated and analyzed by quantitative PCR. The upper band corresponds to the PTP HSC transcript while the lower band corresponds to the triose phosphate isomerase (TPI) internal standard. **B.** lin^{lo}CD34^{hi}sca^{hi} hematopoietic progenitor/stem cells were purified from murine fetal liver and incubated for up to 14 days in IL-s, IL-s, EPO and GM-CSF. RNA was isolated at various times and analyzed by quantitative PCR as described in **A.**

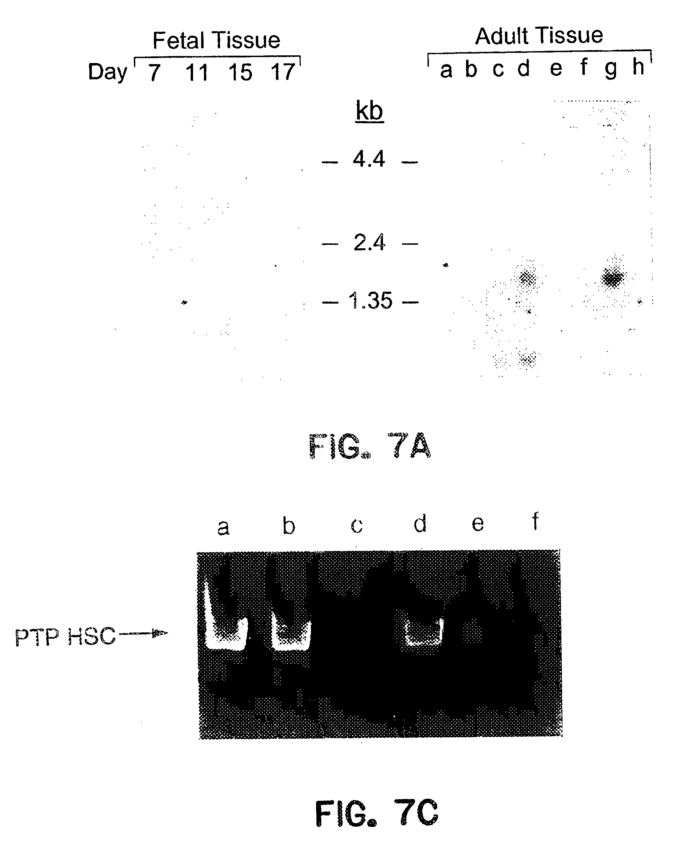

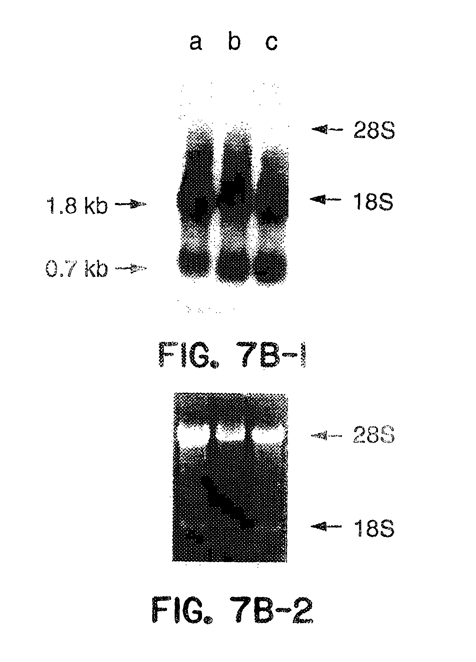

**Figure 7. PTP HSC Transcript analysis in embryonic and adult tissues and hematopoietic cell lines. A.** Illustrated is a tissue northern blot probed with a cDNA encoding PTP HSC. The left panel illustrates RNA isolated from variously aged embryos, while the right panel illustrates RNA isolated from: **a**. heart, **b.**, brain, **c.** spleen, **d.** lung, **e.** liver, **f.** skeletal muscle, **g.** kidney, h. testis. **B.** Illustrated is a northern blot of RNA isolated from BAF 3 (a), 32D (b) and FDCP (c) hematopoietic progenitor cells. Also shown is the ethidium bromide stain of the same gel prior to transfer. **C.** PCR analysis of RNA isolated from BAF 3 (a), 32 D (b), T cell clone (c), FDCP (d), 11 day embryos (e) and a control with no reverse transcriptase (f).
**Figure 8. Partial DNA and deduced protein sequence of the human PTP HSC cDNA.** lllustrated is the partial DNA sequence (SEQ. ID. NO: 17 ) and deduced protein sequence (SEQ. ID. NO: 18) of the human PTP HSC cDNA.

### Detailed Description of the Invention

### A. Definitions

The phrases "non-receptor protein tyrosine phosphatase of hematopoietic stem cells", "tyrosine phosphatase of hematopoietic stem cells" and "PTP HSC" are used interchangeably and refer to a native intracellular protein tyrosine phosphatase which (1) is expressed predominantly in early hematopoietic stem and progenitor cells; (2) predominantly lacks expression in adult tissues; (3) comprises an N-terminal tyrosine phosphatase domain, followed by a region rich in serine, threonine, and proline, and a carboxy terminal region of about 15 to 25 amino acids rich in basic amino acid residues; and (4) is capable of tyrosine dephosphorylation in hematopoietic progenitor cells, and functional derivatives of such native tyrosine phosphatase.

The term "native tyrosine phosphatase" in this context refers to a naturally occurring tyrosine phosphatase, having the described properties, of any human or non-human animal species, with or without the initiating methionine, whether purified from native source, synthesized, produced by recombinant DNA technology or by any combination of these and/or other methods. Native PTP HSCs specifically include the native murine and native human HSC proteins (SEQ. ID. NOs: 2 and , respectively).

A "functional derivative" of a polypeptide is a compound having a qualitative biological activity in common with the native polypeptide. Thus, a functional derivative of a native PTP HSC polypeptide is a compound that has a qualitative biological activity in common with a native PTP HSC. "Functional derivatives" include, but are not limited to, fragments of native polypeptides from any animal species (including humans), derivatives of native (human and non-human) polypeptides and their fragments, and peptide and non-peptide analogs of native polypeptides, provided that they have a biological activity in common with a respective native polypeptide. "Fragments" comprise regions within the sequence of a mature native polypeptide. The term "derivative" is used to define amino acid sequence variants, and covalent modifications of a native polypeptide. "Non-peptide analogs" are organic compounds which display substantially the same surface as peptide analogs of the native polypeptides. Thus, the non-peptide analogs of the native PTP HSCs of the present invention are organic compounds which display substantially the same surface as peptide analogs of the native PTP HSCs. Such compounds interact with other molecules in a similar fashion as the peptide analogs, and mimic a biological activity of native PTP HSC of the present invention. The polypeptide functional derivatives of the native PTP HSCs of the present invention preferably have an active N-terminal tyrosine phosphatase domain, retaining a serine residue at a position corresponding to amino acid position 37 in Figure 1, and retaining an active site cysteine residue at a position corresponding to amino acid position 229 in Figure 1; a region rich in serine, threonine, and proline; and a carboxy-terminal region showing at least about 80% sequence homology with the amino acid sequence between positions 430 and 451 in Figure 1. Preferably, such derivatives have at least about 65%, more preferably at least about 75 %, even more preferably at least about 85%, most preferably at least about 95% overall sequence homology with the amino acid sequence shown in Figure 1 (SEQ. ID. NO: 2) or Figure 8 (SEQ. ID. NO: 18) and retain the ability of tyrosine dephosphorylation in hematopoietic progenitor cells.

The term "biological activity" in the context of the definition of functional derivatives is defined as the possession of at least one adhesive, regulatory or effector function qualitatively in common with a native polypeptide (e.g. PTP HSC). The functional derivatives of the native PTP HSCs of the present invention are unified by their qualitative ability of tyrosine dephosphorylation in hematopoietic progenitor cells. In addition, the functional derivatives of the native PTP HSCs herein preferably are capable of downregulating STAT activation.

The term "agonist" is used to refer to peptide and non-peptide analogs of the native PTP HSCs of the present invention and to antibodies specifically binding such native PTP HSCs provided that they retain the qualitative ability of tyrosine dephosphorylation in hematopoietic progenitor cells.

The term "antagonist" is used to refer to a molecule inhibiting the ability of a PTP HSC of the present invention to dephosphorylate tyrosines. Preferred antagonists essentially completely block tyrosine dephosphorylation caused by a PTP HSC.

"Identity" or "homology" with respect to a native polypeptide and its functional derivative is defined herein as the percentage of amino acid residues in the candidate sequence that are identical with the residues of a corresponding native polypeptide, after aligning the sequences and introducing gaps, if necessary, to achieve the maximum percent homology, and not considering any conservative substitutions as part of the sequence identity. Neither N- or C-terminal extensions nor insertions shall be construed as reducing identity or homology. Methods and computer programs for the alignment are well known in the art.

The term "stem cell" is used in the broadest sense to describe cells which are not terminally differentiated and have the ability to divide throughout the lifetime of the organism, yielding some progeny that differentiate and others that remain stem cells, including stem cells of any tissue type, such as the lining of the gut, the epidermal layer of the skin and the blood-forming tissues.

The term "hematopoietic stem cell" is used in the broadest sense to refer to stem cells from which blood cells derive, including pluripotent stem cells, lymphoid and myeloid stem cells.

The term "hematopoietic progenitor cell" refers to the progeny of a pluripotent hematopoietic stem cell which are committed for a particular line of differentiation. These committed progenitor cells are irreversibly determined as ancestors of only one or a few blood cell types, e.g, erythrocytes or granulocytes.

"Hematopoietic growth factors" are growth factors that influence blood cell formation or differentiation *in vivo*, such as EPO, TPO, IL-3, IL-6, stem cell growth factor, M-CSF, G-CSF, GM-CSF, FTL 3 ligand, LIF, etc., unified by their role in mediating protein phosphorylation. The receptors of these growth factors are either transmembrane tyrosine kinases or are members of the cytokine receptor family.

Ordinarily, the terms "amino acid" and "amino acids" refer to all naturally occurring L-α-amino acids. In some embodiments, however, D-amino acids may be present in the polypeptides or peptides of the present invention in order to facilitate conformational restriction. For example, in order to facilitate disulfide bond formation and stability, a D amino acid cysteine may be provided at one or both termini of a peptide functional derivative or peptide antagonist of the native PTP HSC's of the present invention. The amino acids are identified by either the single-letter or three-letter designations:

| | | | | | |
|---|---|---|---|---|---|
| Asp | D | aspartic acid | Ile | I | isoleucine |
| Thr | T | threonine | Leu | L | leucine |
| Ser | S | serine e | Tyr | Y | tyrosine |
| Glu | E | glutamic acid | Phe | F | phenylalanine |
| Pro | P | proline | His | H | histidine |
| Gly | G | glycine | Lys | K | lysine |
| Ala | A | alanine | Arg | R | arginine |
| Cys | C | cysteine | Trp | W | tryptophan |
| Val | V | valine | Gln | Q | glutamine |
| Met | M | methionine | Asn | N | asparagine |

These amino acids may be classified according to the chemical composition and properties of their side chains. They are broadly classified into two groups, charged and uncharged. Each of these groups is divided into subgroups to classify the amino acids more accurately:

### I. Charged Amino Acids

Acidic Residues: aspartic acid, glutamic acid

Basic Residues: lysine, arginine, histidine

### II. Uncharged Amino Acids

Hydrophilic Residues: serine, threonine, asparagine, glutamine

Aliphatic Residues: glycine, alanine, valine, leucine, isoleucine

Non-polar Residues: cysteine, methionine, proline

Aromatic Residues: phenylalanine, tyrosine, tryptophan

The term "amino acid sequence variant" refers to molecules with some differences in their amino acid sequences as compared to a native amino acid sequence.

Substitutional variants are those that have at least one amino acid residue in a native sequence removed and a different amino acid inserted in its place at the same position. The substitutions may be single, where only one amino acid in the molecule has been substituted, or they may be multiple, where two or more amino acids have been substituted in the same molecule.

Insertional variants are those with one or more amino acids inserted immediately adjacent to an amino acid at a particular position in a native sequence. Immediately adjacent to an amino acid means connected to either the α-carboxy or α-amino functional group of the amino acid.

Deletional variants are those with one or more amino acids in the native amino acid sequence removed. Ordinarily, deletional variants will have one or two amino acids deleted in a particular region of the molecule.

"Antibodies (Abs)" and "immunoglobulins (igs)" are glycoproteins having the same structural characteristics. While antibodies exhibit binding specificity to a specific antigen, immunoglobulins include both antibodies and other antibody-like molecules which lack antigen specificity. Polypeptides of the latter kind are, for example, produced at low levels by the lymph system and at increased levels by myelomas.

Native antibodies and immunoglobulins are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies between the heavy chains of different immunoglobulin isotypes. Each heavy and light chain also has regularly spaced intrachain disulfide bridges. Each heavy chain has at one end a variable domain (V_{H}) followed by a number of constant domains. Each light chain has a variable domain at one and (V_{L}) and a constant domain at its other end; the constant domain of the light chain is aligned with the first constant domain of the heavy chain, and the light chain variable domain is aligned with the variable domain of the heavy chain. Particular amino acid residues are believed to form an interface between the light and heavy chain variable domains (Clothia *et al*., J. Mol. Biol. 186, 651-663 [1985]; Novomy and Haber, Proc. Natl. Acad. Sci. USA 82, 4592-4596 [1985]).

The term "variable" refers to the fact that certain portions of the variable domains differ extensively in sequence among antibodies and are used in the binding and specificity of each particular antibody for its particular antigen. However, the variability is not evenly distributed through the variable domains of antibodies. It is concentrated in three segments called complementarity determining regions (CDRs) or hypervariable regions both in the light chain and the heavy chain variable domains. The more highly conserved portions of variable domains are called the framework (FR). The variable domains of native heavy and light chains each comprise four FR regions, largely adopting a β-sheet configuration, connected by three CDRs, which form loops connecting, and in some cases forming part of, the β-sheet structure. The CDRs in each chain are held together in close proximity by the FR regions and, with the CDRs from the other chain, contribute to the formation of the antigen binding site of antibodies (see Kabat, E.A. *et al*., Sequences of Proteins of Immunological Interest, National Institute of Health, Bethesda, MD [1991]). The constant domains are not involved directly in binding an antibody to an antigen, but exhibit various effector functions, such as participation of the antibody in antibody-dependent cellular toxicity.

Papain digestion of antibodies produces two identical antigen binding fragments, called Fab fragments, each with a single antigen binding site, and a residual "Fc" fragment, whose name reflects its ability to crystallize readily. Pepsin treatment yields an F(ab')₂ fragment that has two antigen combining sites and is still capable of cross-linking antigen.

"Fv" is the minimum antibody fragment which contains a complete antigen recognition and binding site. This region consists of a dimer of one heavy and one light chain variable domain in tight, non-covalent association. It is in this configuration that the three CDRs of each variable domain interact to define an antigen binding site on the surface of the V_{H}-V_{L} dimer. Collectively, the six CDRs confer antigen binding specificity to the antibody. However, even a single variable domain (or half of an Fv comprising only three CDRs specific for an antigen) has the ability to recognize and bind antigen, although at a lower affinity than the entire binding site.

The Fab fragment also contains the constant domain of the light chain and the first constant domain (CH1) of the heavy chain. Fab' fragments differ from Fab fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear a free thiol group. F(ab')₂ antibody fragments originally were produced as pairs of Fab' fragments which have hinge cysteines between them. Other, chemical couplings of antibody fragments are also known.

The light chains of antibodies (immunoglobulins) from any vertebrate species can be assigned to one of two clearly distinct types, called kappa and lambda (λ), based on the amino acid sequences of their constant domains.

Depending on the amino acid sequence of the constant domain of their heavy chains, immunoglobulins can be assigned to different classes. There are five major classes of immunoglobulins: lgA, IgD, IgE, IgG and IgM, and several of these may be further divided into subclasses (isotypes), e.g. IgG-1, IgG-2, IgG-3, and IgG-4; IgA-1 and IgA-2. The heavy chain constant domains that correspond to the different classes of immunoglobulins are called α, delta, epsilon, γ, and µ, respectively. The subunit structures and three-dimensional configurations of different classes of immunoglobulins are well known.

The term "antibody" is used in the broadest sense and specifically covers single monoclonal antibodies (including agonist and antagonist antibodies), antibody compositions with polyepitopic specificity, as well as antibody fragments (e.g., Fab, F(ab')₂, and Fv), so long as they exhibit the desired biological activity.

The term "monoclonal antibody" as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. In addition to their specificity, the monoclonal antibodies are advantageous in that they are synthesized by the hybridoma culture, uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler & Milstein, Nature 256:495 (1975), or may be made by recombinant DNA methods [see, e.g. U.S. Patent No. 4,816,567 (Cabilly *et al*.)].

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567 (Cabilly *et al*.; Morrison *et al*., Proc. Natl. Acad. Sci. USA 81, 6851-6855 [1984]).

"Humanized" forms of non-human (e.g. murine) antibodies are chimeric immunoglobulins, immunoglobulin chains or fragments thereof (such as Fv, Fab, Fab', F(ab')₂ or other antigen-binding subsequences of antibodies) which contain minimal sequence derived from non-human immunoglobulin. For the most part, humanized antibodies are human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Furthermore, humanized antibody may comprise residues which are found neither in the recipient antibody nor in the imported CDR or framework sequences. These modifications are made to further refine and optimize antibody performance. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin. For further details see: Jones *et al*., Nature 321, 522-525 [1986]; Reichmann *et al*., Nature 332, 323-329 [1988]; EP-B-239 400 published 30 September 1987; Presta, Curr. Op. Struct. Biol. 2 593-596 [1992]; and EP-B-451 216 published 24 January 1996).

In the context of the present invention the expressions "cell", "cell line", and "cell culture" are used interchangeably, and all such designations include progeny. It is also understood that all progeny may not be precisely identical in DNA content, due to deliberate or inadvertent mutations. Mutant progeny that have the same function or biological property, as screened for in the originally transformed cell, are included.

The terms "replicable expression vector" and "expression vector" refer to a piece of DNA, usually double-stranded, which may have inserted into it a piece of foreign DNA. Foreign DNA is defined as heterologous DNA, which is DNA not naturally found in the host cell. The vector is used to transport the foreign or heterologous DNA into a suitable host cell. Once in the host cell, the vector can replicate independently of the host chromosomal DNA, and several copies of the vector and its inserted (foreign) DNA may be generated. In addition, the vector contains the necessary elements that permit translating the foreign DNA into a polypeptide. Many molecules of the polypeptide encoded by the foreign DNA can thus be rapidly synthesized.

"Oligonucleotides" are short-length, single- or double-stranded polydeoxynucleotides that are chemically synthesized by known methods [such as phosphotriester, phosphite, or phosphoramidite chemistry, using solid phase techniques such as those described in EP 266,032, published 4 May 1988, or via deoxynucleoside H-phosphanate intermediates as described by Froehler *et al*., Nucl. Acids Res. 14, 5399 (1986). They are then purified on polyacrylamide gels.

### B. Production of PTP HSCs by recombinant DNA technology

### 1. Identification and isolation of nucleic acid encoding PTP HSCs

The native PTP HSC proteins of the present invention may be isolated from relatively undifferentiated, early hematopoietic stem or progenitor cells. The isolation of murine PTP HSC from the CD34^{hi} fraction of murine 10.5 day yolk sac or embryo cells is illustrated in the examples. Similarly, murine PTP HSC can be isolated from CD34^{hi} population originated from bone marrow or fetal liver. The purity of these murine cells was found to be a critical step in isolating the mRNA encoding the new murine PTP HSC of the present invention. A high degree of purity was achieved by purification with a rabbit anti-murine CD34 antibody followed by a lineage depletion step and a positive selection step with the Sca antibody. Alternatively, murine PTP HSC can be detected and obtained from other relatively undifferentiated precursors of mature murine hematopoietic cells, such as, BAF 3, 32D and FDCP hematopoietic progenitor cells, available from the American Type Culture Collection (ATCC). Native human PTP HSC can, for example, be identified in and obtained from human CMK progenitor cells. As the PTP HSCs enzymes have an extremely low abundance in embryonic tissues, their purification by traditional methods would be very cumbersome and inefficient. Instead, cDNA or genomic clones encoding the PTP HSC proteins of the present invention can be prepared using standard techniques of recombinant DNA technology. For example, cDNA library can be constructed by obtaining polyadenylated mRNA from a cell line known to express the desired PTP HSC, and using the mRNA as a template to synthesize double stranded cDNA. Exemplary human and non-human cell lines suitable for this purpose have been listed hereinabove. A PTP HSC polypeptide gene can also be obtained from a genomic library, such as a human genomic cosmid library.

Libraries, either cDNA or genomic, are then screened with probes designed to identify the gene of interest or the protein encoded by it. For cDNA expression libraries, suitable probes include monoclonal and polyclonal antibodies that recognize and specifically bind to a PTP HSC polypeptide. For cDNA libraries, suitable probes include carefully selected oligonucleotide probes (usually of about 20-80 bases in length) that encode known or suspected portions of a PTP HSC polypeptide from the same or different species, and/or complementary or homologous cDNAs or fragments thereof that encode the same or a similar gene. Appropriate probes for screening genomic DNA libraries include, without limitation, oligonucleotides, cDNAs, or fragments thereof that encode the same or a similar gene, and/or homologous genomic DNAs or fragments thereof. Screening the cDNA or genomic library with the selected probe may be conducted using standard procedures as described in Chapters 10-12 of Sambrook *et al*., Molecular Cloning: A Laboratory Manual, New York, Cold Spring Harbor Laboratory Press, 1989.

If DNA encoding an enzyme of the present invention is isolated by using carefully selected oligonucleotide sequences to screen cDNA libraries from various tissues, the oligonucleotide sequences selected as probes should be sufficient in length and sufficiently unambiguous that false positives are minimized. The actual nucleotide sequence(s) is/are usually designed based on regions which have the least codon redundance. The oligonucleotides may be degenerate at one or more positions. The use of degenerate oligonucleotides is of particular importance where a library is screened from a species in which preferential codon usage is not known.

The oligonucleotide must be labeled such that it can be detected upon hybridization to DNA in the library being screened. The preferred method of labeling is to use ATP (e.g., γ³²P) and polynucleotide kinase to radiolabel the 5' end of the oligonucleotide. However, other methods may be used to label the oligonucleotide, including, but not limited to, biotinylation or enzyme labeling.

cDNAs encoding PTP HSCs can also be identified and isolated by other known techniques of recombinant DNA technology, such as by direct expression cloning, or by using the polymerase chain reaction (PCR) as described in U.S. Patent No. 4,683,195, issued 28 July 1987, in section 14 of Sambrook *et al*., *supra*, or in Chapter 15 of Current Protocols in Molecular Biology, Ausubel *et al*. eds., Greene Publishing Associates and Wiley-Interscience 1991. The use of the PCR technique for obtaining cDNA encoding murine PTP HSC or the PTP domain of this native protein is also illustrated in the examples.

Once cDNA encoding a PTP HSC enzyme from one species has been isolated, cDNAs from other species can also be obtained by cross-species hybridization. According to this approach, human or other mammalian cDNA or genomic libraries are probed by labeled oligonucleotide sequences selected from known PTP HSC sequences (such as murine PTP HSC) in accord with known criteria, among which is that the sequence should be sufficient in length and sufficiently unambiguous that false positives are minimized. Typically, a ³²P-labeled oligonucleotide having about 30 to 50 bases is sufficient, particularly if the oligonucleotide contains one or more codons for methionine or tryptophan. Isolated nucleic acid will be DNA that is identified and separated from contaminant nucleic acid encoding other polypeptides from the source of nucleic acid. Hybridization is preferably performed under "stringent conditions" which means (1) employing low ionic strength and hgh temperature for washing, for example, 0.015 sodium chloride/0.0015 M sodium citrate/0.1 % sodium dodecyl sulfate at 50 ° C, or (2) employing during hybridization a denaturing agent, such as formamide, for example, 50% (vol/vol) formamide with 0.1% bovine serum albumin/0. 1% polyvinylpyrrolidone/50 nM sodium phosphate buffer at pH 6.5 with 650 mM sodium chloride, 75 mM sodium citrate at 42 ° C. Another example is the use of 5)% formamide, 5 x SSC (0.75 M sodium chloride, 0.075 M sodium citrate), 50 mM sodium phosphate (pH 6.8), 0.1% sodium pyrophosphate, 5 x Denhardt's solution, sonicated salmon sperm DNA (50 µg/ml), 0.1% SDS, and 10% dextran sulfate at 42 ° C, with washes at 42 ° C in 0.2 x SSC and 0.1% SDS.

Once the sequence is known, the gene encoding a particular PTP HSC polypeptide can also be obtained by chemical synthesis, following one of the methods described in Engels and Uhlmann, Agnew. Chem. Int. Ed. Engl. 28, 716 (1989). These methods include triester, phosphite, phosphoramidite and H-phosphonate methods, PCR and other autoprimer methods, and oligonucleotide syntheses on solid supports.

### 2. Cloning and expression of nucleic acid encoding PTP HSCs

Once the nucleic acid encoding PTP HSC is available, it is generally ligated into a replicable expression vector for further cloning (amplification of the DNA), or for expression.

Expression and cloning vectors are well known in the art and contain a nucleic acid sequence that enables the vector to replicate in one or more selected host cells. The selection of the appropriate vector will depend on 1) whether it is to be used for DNA amplification or for DNA expression, 2) the size of the DNA to be inserted into the vector, and 3) the host cell to be transformed with the vector. Each vector contains various components depending on its function (amplification of DNA of expression of DNA) and the host cell for which it is compatible. The vector components generally include, but are not limited to, one or more- of the following: a signal sequence, an origin of replication, one or more marker genes, an enhancer element, a promoter, and a transcription termination sequence. Construction of suitable vectors containing one or more of the above listed components, the desired coding and control sequences, employs standard ligation techniques. Isolated plasmids or DNA fragments are cleaved, tailored, and religated in the form desired to generate the plasmids required. For analysis to confirm correct sequences in plasmids constructed, the ligation mixtures are commonly used to transform E. coli cells, e.g. E. coli K12 strain 294 (ATCC 31,446) and successful transformants selected by ampicillin or tetracycline resistance where appropriate. Plasmids from the transformants are prepared, analyzed by restriction endonuclease digestion, and/or sequenced by the method of Messing *et al*., Nucleic Acids Res. 9, 309 (1981) or by the method of Maxam *et al*., Methods in Enzymology 65, 499 (1980).

The polypeptides of the present invention may be expressed in a variety of prokaryotic and eukaryotic host cells. Suitable prokaryotes include gram negative or gram positive organisms, for example E. coli or bacilli. A preferred cloning host is E. coli 294 (ATCC 31,446) although other gram negative or gram positive prokaryotes such as E. coli B, E. coli X1776 (ATCC 31,537), E. coli W3110 (ATCC 27,325), Pseudomonas species, or Serratia Marcesans are suitable.

In addition to prokaryotes, eukaryotic microbes such as filamentous fungi or yeast are suitable hosts for vectors herein. Saccharomyces cerevisiae, or common baker's yeast, is the most commonly used among lower eukaryotic host microorganisms. However, a number of other genera, species and strains are commonly available and useful herein, such as S. pombe [Beach and Nurse, Nature 290, 140 (1981)], Kluyveromyces lactis [Louvencourt *et al*., J. Bacteriol. 737 (1983)]; yarrowia (EP 402,226); Pichia pastoris (EP 183,070), Trichoderma reesia (EP 244,234), Neurospora crassa [Case *et al*., Proc. Natl. Acad. Sci. USA 76, 5259-5263 (1979)]; and Aspergillus hosts such as A. nidulans [Ballance *et al*., Biochem. Biophys. Res. Commun. 112, 284-289 (1983); Tilburn *et al*., Gene 26, 205-221 (1983); Yelton *et al*., Proc. Natl. Acad. Sci. USA 81, 1470-1474 (1984)] and A. niger [Kelly and Hynes, EMBO J. 4, 475-479 (1985)].

Suitable host cells may also derive from multicellular organisms. Such host cells are capable of complex processing and glycosylation activities. In principle, any higher eukaryotic cell culture is workable, whether from vertebrate or invertebrate culture, although cells from mammals such as humans are preferred. Examples of invertebrate cells include plants and insect cells. Numerous baculoviral strains and variants and corresponding permissive insect host cells from hosts such as Spodoptera frugiperda (caterpillar), Aedes aegypti (mosquito), Aedes albopictus (mosquito), Drosophila melangaster (fruitfly), and Bombyx mori host cells have been identified. See, e.g. Luckow *et al*., Bio/Technology 6, 47-55 (1988); Miller *et al*., in Genetic Engineering, Setlow, J.K, *et al*., eds., Vol. 8 (Plenum Publishing, 1986), pp. 277-279; and Maeda *et al*., Nature 315, 592-594 (1985). A variety of such viral strains are publicly available, e.g. the L-1 variant of Autographa californica NPV. and such viruses may be used as the virus herein according to the present invention, particularly for transfection of Spodoptera frugiperda cells.

Plant cell cultures of cotton, corn, potato, soybean, petunia, tomato, and tobacco can be utilized as hosts. Typically, plant cells are transfected by incubation with certain strains of the bacterium Agrobacterium tumefaciens, which has been previously manipulated to contain the PTP HSC DNA. During incubation of the plant cell culture with A. tumefaciens, the DNA encoding a PTP HSC is transferred to the plant cell host such that it is transfected, and will, under appropriate conditions, express the PTP HSC DNA. In addition, regulatory and signal sequences compatible with plant cells are available, such as the nopaline synthase promoter and polyadenylation signal sequences. Depicker *et al*., J. Mol. Appl. Gen. 1, 561 (1982). In addition, DNA segments isolated from the upstream region of the T-DNA 780 gene are capable of activating or increasing transcription levels of plant-expressible genes in recombinant DNA-containing plant tissue. See EP 321,196 published 21 June 1989.

However, interest has been greatest in vertebrate cells, and propagation of vertebrate cells in culture (tissue culture) is per se well known. See Tissue Culture, Academic Press, Kruse and Patterson, editors (1973). Examples of useful mammalian host cell lines are monkey kidney CV1 line transformed by SV40 (COS-7, ATCC CRL 1651 ); human embryonic kidney cell line [293 or 293 cells subcloned for growth in suspension culture, Graham *et al*., J. Gen. Virol. 36, 59 (1977)]; baby hamster kidney cells 9BHK, ATCC CCL 10); Chinese hamster ovary cells/-DHFR [CHO, Urlaub and Chasin, Proc. Natl. Acad. Sci. USA 77, 4216 (1980)]; mouse sertolli cells [TM4, Mather, Biol. Reprod. 23, 243-251 (1980)]; monkey kidney cells (CV1 ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL75); human liver cells (Hep G2, HB 8065); mouse mammary tumor (MMT 060562, ATCC CCL51); TRI cells [Mather *et al*., Annals N.Y. Acad. Sci. 383, 44068 (1982)]; MRC 5 cells; FS4 cells; and a human hepatoma cell line (Hep G2). Preferred host cells are human embryonic kidney 293 and Chinese hamster ovary cells.

Particularly useful in the practice of this invention are expression vectors that provide for the transient expression in mammalian cells of DNA encoding a PTP HSC. In general, transient expression involves the use of an expression vector that is able to replicate efficiently in a host cell, such that the host cell accumulates many copies of the expression vector and, in turn, synthesizes high levels of a desired polypeptide encoded by the expression vector. Transient systems, comprising a suitable expression vector and a host cell, allow for the convenient positive identification of polypeptides encoded by clones DNAs, as well as for the rapid screening of such polypeptides for desired biological or physiological properties. Thus, transient expression systems are particularly useful in the invention for purposes of identifying analogs and variants of a PTP HSC.
Other methods, vectors, and host cells suitable for adaptation to the synthesis of the PTP HSC polypeptides in recombinant vertebrate cell culture are described in Getting *et al*., Nature 293, 620-625 (1981); Mantel *et al*., Nature 281, 40-46 (1979); Levinson *et al*.; EP 117,060 and EP 117,058. Particularly useful plasmids for mammalian cell culture expression of the PTP HSC polypeptides are pRK5 (EP 307,247), or pSV16B (PCT Publication No. WO 91/08291).

Other cloning and expression vectors suitable for the expression of the PTP HSCs of the present invention in a variety of host cells are, for example, described in EP 457,758 published 27 November 1991. A large variety of expression vectors is now commercially available. An exemplary commercial yeast expression vector is pPIC.9 (Invitrogen), while an commercially available expression vector suitable for transformation of E. coli cells is PET15b (Novagen).

### C. Culturing the Host Cells

Prokaryotes cells used to produced the PTP HSCs of this invention are cultured in suitable media as describe generally in Sambrook *et al*., supra.

Mammalian cells can be cultured in a variety of media. Commercially available media such as Ham's F10 (Sigma), Minimal Essential Medium (MEM, Sigma), RPMI-1640 (Sigma), and Dulbecco's Modified Eagle's Medium (DMEM, Sigma) are suitable for culturing the host cells. In addition, any of the media described in Ham and Wallace, Meth. Enzymol. 58, 44 (1979); Bames and Sato, Anal. Biochem. 102, 255 (1980), US 4,767,704; 4,657,866; 4,927,762; or 4,560,655; WO 90/03430; WO 87/00195 or US Pat. Re. 30,985 may be used as culture media for the host cells. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleosides (such as adenosine and thymidine), antibiotics (such as Gentamycin^{TM} drug) trace elements (defined as inorganic compounds usually present at final concentrations in the micromolar range), and glucose or an equivalent energy source. Any other necessary supplements may also be included at appropriate concentrations that would be known to those skilled in the art. The culture conditions, such as temperature, pH and the like, suitably are those previously used with the host cell selected for cloning or expression, as the case may be, and will be apparent to the ordinary artisan.

The host cells referred to in this disclosure encompass cells in *in vitro* cell culture as well as cells that are within a host animal or plant.

It is further envisioned that the PTP HSCs of this invention may be produced by homologous recombination, or with recombinant production methods utilizing control elements introduced into cells already containing DNA encoding the particular PTP HSC.

### D. Detecting Gene Amplification/Expression

Gene amplification and/or expression may be measured in a sample directly, for example, by conventional Southern blotting, Northern blotting to quantitate the transcription of mRNA [Thomas, Proc. Natl. Acad. Sci. USA 77, 5201-5205 (1980)], dot blotting (DNA analysis), or *in situ* hybridization, using an appropriately labeled probe, based on the sequences provided herein. Various labels may be employed. most commonly radioisotopes, particularly ³²P. However, other techniques may also be employed, such as using biotin-modified nucleotides for introduction into a polynucleotide. The biotin then serves as a site for binding to avidin or antibodies, which may be labeled with a wide variety of labels, such as radionuclides, fluorescers, enzymes, or the like. Alternatively, antibodies may be employed that can recognize specific duplexes, including DNA duplexes, RNA duplexes, and DNA-RNA hybrid duplexes or DNA-protein duplexes. The antibodies in turn may be labeled and the assay may be carried out where the duplex is bound to the surface, so that upon the formation of duplex on the surface, the presence of antibody bound to the duplex can be detected.

Gene expression, alternatively, may be measured by immunological methods, such as immunohistochemical staining of tissue sections and assay of cell culture or body fluids, to quantitate directly the expression of gene product. With immunohistochemical staining techniques, a cell sample is prepared, typically by dehydration and fixation, followed by reaction with labeled antibodies specific for the gene product coupled, where the labels are usually visually detectable, such as enzymatic labels, fluorescent labels, luminescent labels, and the like. A particularly sensitive staining technique suitable for use in the present invention is described by Hse *et al*., Am. J. Clin. Pharm. 75, 734-738 (1980).

Antibodies useful for immunohistochemical staining and/or assay of sample fluids may be either monoclonal or polyclonal, and may be prepared in any animal. Conveniently, the antibodies may be prepared against a native PTP HSC polypeptide, or against a synthetic peptide based on the DNA sequence provided herein as described further hereinbelow.

### E. Amino Acid Sequence Variants of a native PTP HSCs

Amino acid sequence variants of native PTP HSCs are prepared by methods known in the art by introducing appropriate nucleotide changes into a PTP HSC DNA, or by *in vitro* synthesis of the desired polypeptide. There are two principal variables in the construction of amino acid sequence variants: the location of the mutation site and the nature of the mutation. With the exception of naturally-occurring alleles, which do not require the manipulation of the DNA sequence encoding the PTP HSC, the amino acid sequence variants of PTP HSCs are preferably constructed by mutating the DNA, either to arrive at an allele or an amino acid sequence variant that does not occur in nature.

One group of the mutations will be created within the phosphatase (PTP) domain of the enzymes of the present invention. Non-conservative substitutions within this domain may result in PTP HSC variants which loose their ability to dephosphatase tyrosines and will, therefore, be useful as antagonists of native PTP HSCs. PTP HSC variants mutated to enhance their enzymatic activity will be useful, for example, as more effective inhibitors of progenitor/stem cell differentiation.

Alternatively or in addition, amino acid alterations can be made at sites that differ in PTP HSC proteins from various species, or in highly conserved regions, depending on the goal to be achieved. Sites at such locations will typically be modified in series, e.g. by (1) substituting first with conservative choices and then with more radical selections depending upon the results achieved, (2) deleting the target residue or residues, or (3) inserting residues of the same or different class adjacent to the located site, or combinations of options 1-3. One helpful technique is called "alanine scanning" (Cunningham and Wells, Science 244, 1081-1085 [1989]).

After identifying the desired mutation(s), the gene encoding a PTP HSC variant can, for example, be obtained by chemical synthesis as hereinabove described. More preferably, DNA encoding a PTP HSC amino acid sequence variant is prepared by site-directed mutagenesis of DNA that encodes an earlier prepared variant or a nonvariant version of the PTP HSC. Site-directed (site-specific) mutagenesis allows the production of PTP HSC variants through the use of specific oligonucleotide sequences that encode the DNA sequence of the desired mutation, as well as a sufficient number of adjacent nucleotides, to provide a primer sequence of sufficient size and sequence complexity to form a stable duplex on both sides of the deletion junction being traversed. Typically, a primer of about 20 to 25 nucleotides in length is preferred, with about 5 to 10 residues on both sides of the junction of the sequence being altered. In general, the techniques of site-specific mutagenesis are well known in the art, as exemplified by publications such as, Edelman *et al*., DNA 2, 183 (1983). As will be appreciated, the site-specific mutagenesis technique typically employs a phage vector that exists in both a single-stranded and double-stranded form. Typical vectors useful in site-directed mutagenesis include vectors such as the M13 phage, for example, as disclosed by Messing *et al*., Third Cleveland Symposium on Macromolecules and Recombinant DNA, A. Walton, ed., Elsevier, Amsterdam (1981). This and other phage vectors are commercially available and their use is well known to those skilled in the art. A versatile and efficient procedure for the construction of oligodeoxyribonucleotide directed site-specific mutations in DNA fragments using M 13 - derived vectors was published by Zoller, M.J. and Smith, M., Nucleic Acids Res. 10, 6487-6500 [1982]). Also. plasmid vectors that contain a single-stranded phage origin of replication (Veira *et al*., Meth. Enzymol. 153, 3 [ 1987]) may be employed to obtain single-stranded DNA. Alternatively, nucleotide substitutions are introduced by synthesizing the appropriate DNA fragment *in vitro,* and amplifying it by PCR procedures known in the art.

The PCR technique may also be used in creating amino acid sequence variants of a PTP HSC. In a specific example of PCR mutagenesis, template plasmid DNA (1 1 µg) is linearized by digestion with a restriction endonuclease that has a unique recognition site in the plasmid DNA outside of the region to be amplified. Of this material, 100 ng is added to a PCR mixture containing PCR buffer, which contains the four deoxynucleotide triphosphates and is included in the GeneAmp^{R} kits (obtained from Perkin-Elmer Cetus, Norwalk, CT and Emeryville, CA), and 25 pmole of each oligonucleotide primer, to a final volume of 50 µl. The reaction mixture is overlayered with 35 µl mineral oil. The reaction is denatured for 5 minutes at 100°C, placed briefly on ice, and then 1 µl Thermus aquaticus (Taq) DNA polymerase (5 units/ 1), purchased from Perkin-Elmer Cetus, Norwalk, CT and Emeryville, CA) is added below the mineral oil layer. The reaction mixture is then inserted into a DNA Thermal Cycler (purchased from Perkin-Elmer Cetus) programmed as follows:
2 min. 55°C,
30 sec. 72°C, then 19 cycles of the following:
30 sec. 94°C,
30 sec. 55°C, and
30 sec. 72°C.

At the end of the program, the reaction vial is removed from the thermal cycler and the aqueous phase transferred to a new vial, extracted with phenol/chloroform (50:50 vol), and ethanol precipitated, and the DNA is recovered by standard procedures. This material is subsequently subjected to appropriate treatments for insertion into a vector.

Another method for preparing variants, cassette mutagenesis, is based on the technique described by Wells *et al*. [Gene 34, 315 (1985)].

Additionally, the so-called phagemid display method may be useful in making amino acid sequence variants of native or variant PTP HSCs or their fragments. This method involves (a) constructing a replicable expression vector comprising a first gene encoding an receptor to be mutated, a second gene encoding at least a portion of a natural or wild-type phage coat protein wherein the first and second genes are heterologous, and a transcription regulatory element operably linked to the first and second genes, thereby forming a gene fusion encoding a fusion protein; (b) mutating the vector at one or more selected positions within the first gene thereby forming a family of related plasmids; (c) transforming suitable host cells with the plasmids; (d) infecting the transformed host cells with a helper phage having a gene encoding the phage coat protein; (e) culturing the transformed infected host cells under conditions suitable for forming recombinant phagemid particles containing at least a portion of the plasmid and capable of transforming the host, the conditions adjusted so that no more than a minor amount of phagemid particles display more than one copy of the fusion protein on the surface of the particle; (f) contacting the phagemid particles with a suitable antigen so that at least a portion of the phagemid particles bind to the antigen; and (g) separating the phagemid particles that bind from those that do not. Steps (d) through (g) can be repeated one or more times. Preferably in this method the plasmid is under tight control of the transcription regulatory element, and the culturing conditions are adjusted so that the amount or number of phagemid particles displaying more than one copy of the fusion protein on the surface of the particle is less than about 1%. Also, preferably, the amount of phagemid particles displaying more than one copy of the fusion protein is less than 10% of the amount of phagemid particles displaying a single copy of the fusion protein. Most preferably, the amount is less than 20%. Typically in this method, the expression vector will further contain a secretory signal sequence fused to the DNA encoding each subunit of the polypeptide and the transcription regulatory element will be a promoter system. Preferred promoter systems are selected from lac Z, λ_{PL}, tac, T7 polymerase, tryptophan, and alkaline phosphatase promoters and combinations thereof. Also, normally the method will employ a helper phage selected from M13K07, M13R408, M13-VCS, and Phi X 174. The preferred helper phage is M13K07, and the preferred coat protein is the M13 Phage gene III coat protein. The preferred host is *E. coli*, and protease-deficient strains of *E. coli*.

Further details of the foregoing and similar mutagenesis techniques are found in general textbooks, such as, for example, Sambrook *et al*., *supra,* and Current Protocols in Molecular Biology, Ausubel *et al.* eds., *supra,*

Naturally-occurring amino acids are divided into groups based on common side chain properties:
(1) hydrophobic: norleucine, met, ala, val, leu, ile;
(2) neutral hydrophobic: cys, ser, thr;
(3) acidic: asp, glu;
(4) basic: asn, gin, his, lys, arg;
(5) residues that influence chain orientation: gly, pro; and
(6) aromatic: trp, tyr, phe.

Conservative substitutions involve exchanging a member within one group for another member within the same group, whereas non-conservative substitutions will entail exchanging a member of one of these classes for another.

Amino acid sequence deletions generally range from about 1 to 30 residues, more preferably about 1 to 10 residues, and typically are contiguous.

Amino acid insertions include amino- and/or carboxyl-terminal fusions ranging in length from one residue to polypeptides containing a hundred or more residues, as well as intrasequence insertions of single or multiple amino acid residues. intrasequence insertions (i.e. insertions within the PTP HSC protein amino acid sequence) may range generally from about 1 to 10 residues, more preferably 1 to 5 residues, more preferably 1 to 3 residues. Examples of terminal insertions include the PTP HSC polypeptides with an N-terminal methionyl residue, an artifact of its direct expression in bacterial recombinant cell culture, and fusion of a heterologous N-terminal signal sequence to the N-terminus of the PTP HSC molecule to facilitate the secretion of the mature PTP HSC from recombinant host cells. Such signal sequences will generally be obtained from, and thus homologous to, the intended host cell species. Suitable sequences include STII or Ipp for E. coli, alpha factor for yeast, and viral signals such as herpes gD for mammalian cells.

Other insertional variants of the native PTP HSC molecules include the fusion of the N- or C-terminus of the TRAF molecule to immunogenic polypeptides, e.g. bacterial polypeptides such as beta-lactamase or an enzyme encoded by the E. coli trp locus, or yeast protein, and C-terminal fusions with proteins having a long half life such as immunoglobulin regions (preferably immunoglobulin constant regions), albumin, or ferritin, as described in WO 89/02922 published on 6 April 1989.

Since it is often difficult to predict in advance the characteristics of a variant PTP HSC, it will be appreciated that some screening will be needed to select the optimum variant.

### F. Covalent Modifications of PTP HSC Polypeptides

Covalent modifications of PTP HSCs are included within the scope herein. Such modifications are traditionally introduced by reacting targeted amino acid residues of the PTP HSC polypeptides with an organic derivatizing agent that is capable of reacting with selected sides or terminal residues, or by harnessing mechanisms of post-translational modifications that function in selected recombinant host cells. The resultant covalent derivatives are useful in programs directed at identifying residues important for biological activity, for immunoassays of the PTP HSC, or for the preparation of anti-PTP HSC antibodies for immunoaffinity purification of the recombinant. For example, complete inactivation of the biological activity of the protein after reaction with ninhydrin would suggest that at least one arginyl or lysyl residue is critical for its activity, whereafter the individual residues which were modified under the conditions selected are identified by isolation of a peptide fragment containing the modified amino acid residue. Such modifications are within the ordinary skill in the art and are performed without undue experimentation.

Cysteinyl residues most commonly are reacted with α-haloacetates (and corresponding amines), such as chloroacetic acid or chloroacetamide, to give carboxymethyl or carboxyamidomethyl derivatives. Cysteinyl residues also are derivatized by reaction with bromotrifluoroacetone, α-bromo-β-(5-imidozoyl)propionic acid, chloroacetyl phosphate, N-alkylmaleimides, 3-nitro-2-pyridyl disulfide, methyl 2-pyridyl disulfide, p-chloromercuribenzoate, 2-chloromercuri-4-nitrophenol, or chloro-7-nitrobenzo-2-oxa- 1,3-diazole.

Histidyl residues are derivatized by reaction with diethylpyrocarbonate at pH 5.5-7.0 because this agent is relatively specific for the histidyl side chain. Para-bromophenacyl bromide also is useful; the reaction is preferably performed in 0.1 M sodium cacodylate at pH 6.0.

Lysinyl and amino terminal residues are reacted with succinic or other carboxylic acid anhydrides. Derivatization with these agents has the effect of reversing the charge of the lysinyl residues. Other suitable reagents for derivatizing α-amino-containing residues include imidoesters such as methyl picolinimidate; pyridoxal phosphate; pyridoxal; chloroborohydride; trinitrobenzenesulfonic acid; O-methylisourea; 2,4-pentanedione; and transaminase-catalyzed reaction with glyoxylate.

Arginyl residues are modified by reaction with one or several conventional reagents, among them phenylglyoxal, 2,3-butanedione, 1,2-cyclohexanedione, and ninhydrin. Derivatization of arginine residues requires that the reaction be performed in alkaline conditions because of the high pKₐ of the guanidine functional group. Furthermore, these reagents may react with the groups of lysine as well as the arginine epsilon-amino group.

The specific modification of tyrosyl residues may be made, with particular interest in introducing spectral labels into tyrosyl residues by reaction with aromatic diazonium compounds or tetranitromethane. Most commonly, N-acetylimidizole and tetranitromethane are used to form O-acetyl tyrosyl species and 3-nitro derivatives, respectively. Tyrosyl residues are iodinated using ¹²⁵I or ¹³¹I to prepare labeled proteins for use in radioimmunoassay.

Carboxyl side groups (aspartyl or glutamyl) are selectively modified by reaction with carbodiimides (R'-N=C=N-R') such as 1-cyclohexyl-3-(2-morpholinyl-4-ethyl) carbodiimide or 1-ethyl-3-(4-azonia-4,4-dimethylpentyl) carbodiimide. Furthermore, aspartyl and glutamyl residues are converted to asparaginyl and glutaminyl residues by reaction with ammonium ions.

Glutaminyl and asparaginyl residues are frequently deamidated to the corresponding glutamyl and aspartyl residues. Alternatively, these residues are deamidated under mildly acidic conditions. Either form of these residues falls within the scope of this invention.

Other modifications include hydroxylation of proline and lysine, phosphorylation of hydroxyl groups of seryl, threonyl or tyrosyl residues, methylation of the α-amino groups of lysine, arginine, and histidine side chains (T.E. Creighton, Proteins: Structure and Molecular Properties, W.H. Freeman & Co., San Francisco, pp. 79-86 [1983]), acetylation of the N-terminal amine, and amidation of any C-terminal carboxyl group. The molecules may further be covalently linked to nonproteinaceous polymers, e.g. polyethylene glycol, polypropylene glycol or polyoxyalkylenes, in the manner set forth in U.S.S.N. 07/275,296 or U.S. patents 4,640,835; 4,496,689; 4,301,144; 4,670,417; 4,791,192 or 4,179,337.

Derivatization with bifunctional agents is useful for preparing intramolecular aggregates of the PTP HSCs with polypeptides as well as for cross-linking the PTP HSC polypeptide to a water insoluble support matrix or surface for use in assays or affinity purification. In addition, a study of interchain cross-links will provide direct information on conformational structure. Commonly used cross-linking agents include 1,1-bis(diazoacetyl)-2-phenylethane, glutaraldehyde, N-hydroxysuccinimide esters, homobifunctional imidoesters, and bifunctional maleimides. Derivatizing agents such as methyl-3-[(p-azidophenyl)dithio]propioimidate yield photoactivatable intermediates which are capable of forming cross-links in the presence of light. Alternatively, reactive water insoluble matrices such as cyanogen bromide activated carbohydrates and the systems reactive substrates described in U.S. Patent Nos. 3,959,642; 3,969,287; 3,691,016; 4,195,128; 4,247,642; 4,229,537; 4,055,635; and 4,330,440 are employed for protein immobilization and cross-linking.

Certain post-translational modifications are the result of the action of recombinant host cells on the expressed polypeptide. Glutaminyl and aspariginyl residues are frequently post-translationally deamidated to the corresponding glutamyl and aspartyl residues. Alternatively, these residues are deamidated under mildly acidic conditions. Either form of these residues falls within the scope of this invention.

Other post-translational modifications include hydroxylation of proline and lysine, phosphorylation of hydroxyl groups of seryl, threonyl or tyrosyl residues, methylation of the α-amino groups of lysine, arginine, and histidine side chains [T.E. Creighton, Proteins: Structure and Molecular Properties, W.H. Freeman & Co., San Francisco, pp. 79-86 (1983)].

Other derivatives comprise the novel peptides of this invention covalently bonded to a nonproteinaceous polymer. The nonproteinaceous polymer ordinarily is a hydrophilic synthetic polymer, i.e. a polymer not otherwise found in nature. However, polymers which exist in nature and are produced by recombinant or *in vitro* methods are useful, as are polymers which are isolated from nature. Hydrophilic polyvinyl polymers fall within the scope of this invention, e.g. polyvinylalcohol and polyvinylpyrrolidone. Particularly useful are polyvinylalkylene ethers such a polyethylene glycol, polypropylene glycol.

The PTP HSC polypeptides may be linked to various nonproteinaceous polymers, such as polyethylene glycol, polypropylene glycol or polyoxyalkylenes, in the manner set forth in U.S. Patent Nos. 4,640,835; 4,496,689; 4,301,144; 4,670,417; 4,791,192 or 4,179,337.

The PTP HSCs may be entrapped in microcapsules prepared, for example, by coacervation techniques or by interfacial polymerization, in colloidal drug delivery systems (e.g. liposomes, albumin microspheres, microemulsions, nano-particles and nanocapsules), or in macroemulsions. Such techniques are disclosed in Remington's Pharmaceutical Sciences, 16th Edition, Osol, A., Ed. (1980).

### G. Anti-PTP HSC antibody preparation

### (i) Polyclonal antibodies

Polyclonal antibodies to a PTP HSC molecule generally are raised in animals by multiple subcutaneous (sc) or intraperitoneal (ip) injections of the PTP HSC and an adjuvant. It may be useful to conjugate the PTP HSC or a fragment containing the target amino acid sequence to a protein that is immunogenic in the species to be immunized, e.g. keyhole limpet hemocyanin, serum albumin, bovine thyroglobulin, or soybean trypsin inhibitor using a bifunctional or derivatizing agent, for example maleimidobenzoyl sulfosuccinimide ester (conjugation through cysteine residues), N-hydroxysuccinimide (through lysine residues), glytaraldehyde, succinic anhydride, SOCl₂, or R¹ N=C=NR, where R and R¹ are different alkyl groups.

Animals are immunized against the immunogenic conjugates or derivatives by combining 1 mg or 1 µg of conjugate (for rabbits or mice, respectively) with 3 volumes of Freud's complete adjuvant and injecting the solution intradermally at multiple sites. One month later the animals are boosted with 1/5 to 1/10 the original amount of conjugate in Freud's complete adjuvant by subcutaneous injection at multiple sites. 7 to 14 days later the animals are bled and the serum is assayed for anti-PTP HSC antibody titer. Animals are boosted until the titer plateaus. Preferably, the animal boosted with the conjugate of the same PTP HSC, but conjugated to a different protein and/or through a different cross-linking reagent. Conjugates also can be made in recombinant cell culture as protein fusions. Also, aggregating agents such as alum are used to enhance the immune response.

### (ii) Monoclonal antibodies

Monoclonal antibodies are obtained from a population of substantially homogeneous antibodies, i.e., the individual antibodies comprising the population are identical except for possible naturally-occurring mutations that may be present in minor amounts. Thus, the modifier "monoclonal" indicates the character of the antibody as not being a mixture of discrete antibodies.

For example, the anti-PTP HSC monoclonal antibodies of the invention may be made using the hybridoma method first described by Kohler & Milstein, Nature 256:495 (1975), or may be made by recombinant DNA methods [Cabilly, *et al*., U.S. Pat. No. 4,816,567].

In the hybridoma method, a mouse or other appropriate host animal, such as hamster is immunized as hereinabove described to elicit lymphocytes that produce or are capable of producing antibodies that will specifically bind to the protein used for immunization. Alternatively, lymphocytes may be immunized in vitro. Lymphocytes then are fused with myeloma cells using a suitable fusing agent, such as polyethylene glycol, to form a hybridoma cell [Goding, Monoclonal Antibodies: Principles and Practice, pp.59-103 (Academic Press, 1986)].

The hybridoma cells thus prepared are seeded and grown in a suitable culture medium that preferably contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium), which substances prevent the growth of HGPRT-deficient cells.

Preferred myeloma cells are those that fuse efficiently, support stable high level expression of antibody by the selected antibody-producing cells, and are sensitive to a medium such as HAT medium. Among these, preferred myeloma cell lines are murine myeloma lines, such as those derived from MOPC-21 and MPC- 11 mouse tumors available from the Salk Institute Cell Distribution Center, San Diego, California USA, and SP-2 cells available from the American Type Culture Collection, Rockville, Maryland USA. Human myeloma and mouse-human heteromyeloma cell lines also have been described for the production of human monoclonal antibodies [Kozbor, J. Immunol. 133:3001 (1984); Brodeur, *et al*., Monoclonal Antibody Production Techniques and Applications, pp.51-63 (Marcel Dekker, inc., New York, 1987)].

Culture medium in which hybridoma cells are growing is assayed for production of monoclonal antibodies directed against PTP HSC. Preferably, the binding specificity of monoclonal antibodies produced by hybridoma cells is determined by immunoprecipitation or by an in vitro binding assay, such as radioimmunoassay (RIA) or enzyme-linked immunoabsorbent assay (ELISA). The binding affinity of the monoclonal antibody can, for example, be determined by the Scatchard analysis of Munson & Pollard, Anal. Biochem. 107:220 (1980).

After hybridoma cells are identified that produce antibodies of the desired specificity, affinity, and/or activity, the clones may be subcloned by limiting dilution procedures and grown by standard methods. Goding, Monoclonal Antibodies: Principles and Practice, pp.59-104 (Academic Press, 1986). Suitable culture media for this purpose include, for example, Dulbecco's Modified Eagle's Medium or RPMI-1640 medium. In addition, the hybridoma cells may be grown in vivo as ascites tumors in an animal.

The monoclonal antibodies secreted by the subclones are suitably separated from the culture medium, ascites fluid, or serum by conventional immunoglobulin purification procedures such as, for example, protein A-Sepharose, hydroxylapatite chromatography, gel electrophoresis, dialysis, or affinity chromatography.

DNA encoding the monoclonal antibodies of the invention is readily isolated and sequenced using conventional procedures (e.g., by using oligonucleotide probes that are capable of binding specifically to genes encoding the heavy and light chains of murine antibodies). The hybridoma cells of the invention serve as a preferred source of such DNA. Once isolated, the DNA may be placed into expression vectors, which are then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also may be modified, for example, by substituting the coding sequence for human heavy and light chain constant domains in place of the homologous murine sequences, Morrison, *et al*., Proc. Nat. Acad. Sci. 81, 6851 (1984), or by covalently joining to the immunoglobulin coding sequence all or part of the coding sequence for a non-immunoglobulin polypeptide. In that manner, "chimeric" or "hybrid" antibodies are prepared that have the binding specificity of an anti-TRAF monoclonal antibody herein.

Typically such non-immunoglobulin polypeptides are substituted for the constant domains of an antibody of the invention, or they are substituted for the variable domains of one antigen-combining site of an antibody of the invention to create a chimeric bivalent antibody comprising one antigen-combining site having specificity for a PTP HSC and another antigen-combining site having specificity for a different antigen.

Chimeric or hybrid antibodies also may be prepared in vitro using known methods in synthetic protein chemistry, including those involving crosslinking agents. For example, immunotoxins may be constructed using a disulfide exchange reaction or by forming a thioether bond. Examples of suitable reagents for this purpose include iminothiolate and methyl-4-mercaptobutyrimidate.

For diagnostic applications, the antibodies of the invention typically will be labeled with a detectable moiety. The detectable moiety can be any one which is capable of producing, either directly or indirectly, a detectable signal. For example, the detectable moiety may be a radioisotope, such as ³H, ¹⁴C, ³²P, ³⁵S, or ¹²⁵I, a fluorescent or chemiluminescent compound, such as fluorescein isothiocyanate, rhodamine, or luciferin; biotin; radioactive isotopic labels, such as, e.g., ¹²⁵I, ³²P, ¹⁴ C, or ³H, or an enzyme, such as alkaline phosphatase, beta-galactosidase or horseradish peroxidase.

Any method known in the art for separately conjugating the antibody to the detectable moiety may be employed, including those methods described by Hunter, *et al*., Nature 144:945 (1962); David, *et al*., Biochemistry 13:1014(1974); Pain, *et al*., J. Immunol. Meth. 40:219 (1981); and Nygren, J. Histochem. and Cytochem. 30:407 (1982).

The antibodies of the present invention may be employed in any known assay method, such as competitive binding assays, direct and indirect sandwich assays, and immunoprecipitation assays. Zola, Monoclonal Antibodies: A Manual of Techniques, pp.147-158 (CRC Press, Inc., 1987).

Competitive binding assays rely on the ability of a labeled standard (which may be a PTP HSC polypeptide or an immunologically reactive portion thereof) to compete with the test sample analyte (PTP HSC) for binding with a limited amount of antibody. The amount of PTP HSC in the test sample is inversely proportional to the amount of standard that becomes bound to the antibodies. To facilitate determining the amount of standard that becomes bound, the antibodies generally are insolubilized before or after the competition, so that the standard and analyte that are bound to the antibodies may conveniently be separated from the standard and analyte which remain unbound.

Sandwich assays involve the use of two antibodies, each capable of binding to a different immunogenic portion, or epitope, of the protein to be detected. In a sandwich assay, the test sample analyte is bound by a first antibody which is immobilized on a solid support, and thereafter a second antibody binds to the analyte, thus forming an insoluble three part complex. David & Greene, U.S. Pat No. 4,376,110. The second antibody may itself be labeled with a detectable moiety (direct sandwich assays) or may be measured using an anti-immunoglobulin antibody that is labeled with a detectable moiety (indirect sandwich assay). For example, one type of sandwich assay is an ELISA assay, in which case the detectable moiety is an enzyme.

### (iii) Humanized antibodies

Methods for humanizing non-human antibodies are well known in the art. Generally, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers [Jones *et al*., Nature 321, 522-525 (1986); Riechmann *et al*., Nature 332, 323-327 (1988); Verhoeyen *et al*., Science 239, 1534-1536 (1988)], by substituting rodent CDRs or CDR sequences for the corresponding sequences of a human antibody. Accordingly, such "humanized" antibodies are chimeric antibodies (Cabilly, supra), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

It is important that antibodies be humanized with retention of high affinity for the antigen and other favorable biological properties. To achieve this goal, according to a preferred method, humanized antibodies are prepared by a process of analysis of the parental sequences and various conceptual humanized products using three dimensional models of the parental and humanized sequences. Three dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, i.e. the analysis of residues that influence the ability of the candidate immunoglobulin to bind its antigen. In this way, FR residues can be selected and combined from the consensus and import sequence so that the desired antibody characteristic, such as increased affinity for the target antigen(s), is achieved. In general, the CDR residues are directly and most substantially involved in influencing antigen binding. For further details see U.S. application Serial No. 07/934,373 filed 21 August 1992, which is a continuation-in-part of application Serial No. 07/715,272 filed 14 June 1991.

Alternatively, it is now possible to produce transgenic animals (e.g. mice) that are capable, upon immunization, of producing a full repertoire of human antibodies in the absence of endogenous immunoglobulin production. For example, it has been described that the homozygous deletion of the antibody heavy chain joining region (J_{H}) gene in chimeric and germ-line mutant mice results in complete inhibition of endogenous antibody production. Transfer of the human germ-line immunoglobulin gene array in such germ-line mutant mice will result in the production of human antibodies upon antigen challenge. See, e.g. Jakobovits *et al*., Proc. Natl. Acad. Sci. USA 90, 2551-255 (1993); Jakobovits *et al*., Nature 362, 255-258 (1993).

### (iv) Bispecific antibodies

Bispecific antibodies are monoclonal, preferably human or humanized, antibodies that have binding specificities for at least two different antigens. In the present case, one of the binding specificities is for a PTP HSC, the other one is for any other antigen, for example an antigen expressed on the surface of a leukemia cell, if the antibody is an antagonist of a native PTP HSC and is used to induce differentiation of undifferentiated lekemia cells. If an agonist antibody specifically binding to a native PTP HSC is used to expand stem cells with growth factors, as hereinafter described, the second specificity could be provided by a stem cell growth factor. Such constructs can also be referred to as bispecific immunoadhesins. Methods for making bispecific antibodies (and bispecific immunoadhesins) are known in the art.

Traditionally, the recombinant production of bispecific antibodies is based on the coexpression of two immunoglobulin heavy chain-light chain pairs, where the two heavy chains have different specificities (Millstein and Cuello, Nature 305, 537-539 (1983)). Because of the random assortment of immunoglobulin heavy and light chains, these hybridomas (quadromas) produce a potential mixture of 10 different antibody molecules, of which only one has the correct bispecific structure. The purification of the correct molecule, which is usually done by affinity chromatography steps, is rather cumbersome, and the product yields are low. Similar procedures are disclosed in PCT application publication No. WO 93/08829 (published 13 May 1993), and in Traunecker *et al*., EMBO 10, 3655-3659 (1991).

According to a different and more preferred approach, antibody variable domains with the desired binding specificities (antibody-antigen combining sites) are fused to immunoglobulin constant domain sequences. The fusion preferably is with an immunoglobulin heavy chain constant domain, comprising at least part of the hinge, and second and third constant regions of an immunoglobulin heavy chain (CH2 and CH3). It is preferred to have the first heavy chain constant region (CH 1 ) containing the site necessary for light chain binding, present in at least one of the fusions. DNAs encoding the immunoglobulin heavy chain fusions and, if desired, the immunoglobulin light chain, are inserted into separate expression vectors, and are cotransfected into a suitable host organism. This provides for great flexibility in adjusting the mutual proportions of the three polypeptide fragments in embodiments when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance. In a preferred embodiment of this approach, the bispecific antibodies are composed of hybrid immunoglobulin heavy chain with a first binding specificity in one arm, and a hybrid immunoglobulin heavy chain-light chain pair (providing a second binding specificity) in the other arm. It was found that this asymmetric structure facilitates the separation of the desired bispecific compound from unwanted immunoglobulin chain combinations, as the presence of an immunoglobulin light chain in only one half of the bispecific molecule provides for a facile way of separation. This approach is disclosed in copending application Serial No. 07/931,811 filed 17 August 1992.

For further details of generating bispecific antibodies see, for example, Suresh *et al*., Methods in Enzymology 121, 210 (1986).

### (v) Heteroconjugate antibodies

Heteroconjugate antibodies are also within the scope of the present invention. Heteroconjugate antibodies are composed of two covalently joined antibodies. Such antibodies have, for example, been proposed to target immune system cells to unwanted cells (U.S. Patent No. 4,676,980), and for treatment of HIV infection (PCT application publication Nos. WO 91/00360 and WO 92/200373; EP 03089). Heteroconjugate antibodies may be made using any convenient cross-linking methods. Suitable cross-linking agents are well known in the art, and are disclosed in U.S. Patent No. 4,676,980, along with a number of cross-linking techniques.

### H. Peptide and non-peptide analogs of polypeptide PTP HSCs

Peptide analogs of the PTP HSC polypeptides of the present invention are modelled based upon the three-dimensional structure of the native polypeptides. Peptides may be synthesized by well known techniques such as the solid-phase synthetic techniques initially described in Merrifield, J. Am. Chem. Soc. 15, 2149-2154 (1963). Other peptide synthesis techniques are, for examples, described in Bodanszky *et al*., Peptide Synthesis, John Wiley & Sons, 2nd Ed., 1976, as well as in other reference books readily available for those skilled in the art. A summary of peptide synthesis techniques may be found in Stuart and Young, Solid Phase Peptide Synthelia, Pierce Chemical Company, Rockford, IL (1984). Peptides may also be prepared by recombinant DNA technology, using a DNA sequence encoding the desired peptide.

In addition to peptide analogs, the present invention also contemplates non-peptide (e.g. organic) compounds which display substantially the same surface as the peptide analogs of the present invention, and therefore interact with other molecules in a similar fashion.

### I. Use of the PTP HSCs

The PTP HSCs of the present invention are useful for a variety of purposes. For example, native PTP HSCs are useful for the identification and isolation of a PTP HSC analog in another mammalian species. Native PTP HSCs and their functional equivalents are also useful in screening assays designed to identify agonist of antagonist of native PTP HSCs. Such assays may take the form of any conventional cell-type or biochemical binding assay, and can be performed in a variety of assay formats well known for those skilled in the art. As example is the so called "two-hybrid" assay format using the Matchmaker Two-Hybrid System (Clontech) according to the manufacturer's instructions.

The PTP HSCs of the present invention as well as their agonists can additionally be used for the maintenance of stem/progenitor cells in cell culture. Agonists which inhibit differentiation but allow for hematopoietic stem cell growth are particularly useful for this purpose, since their use results in an amplification of the stem cells without differentiation (self-renewal). This process might be useful, as an example, for the expansion of hematopoietic stem cells prior to autologous or heterologous bone marrow transplantation. The same approach can be used *in vivo* for the expansion of stem cells with growth factors, in the absence of diferentiation.

It is believed that the native PTP HSCs of the present invention may be expressed in leukemic cells. Accordingly, antagonist of the PTP HSCs of the present invention may be used for the induction of differentiation of undifferentiated leukemia cells. This might allow for aggressive undifferentiated leukemia cells to become differentiated, which, in turn, facilitates their treatment.

PTP HSC antagonists may also be used to induce differentiation of hematopoietic stem cells. As inhibition of the native PTP HSC enzyme might induce progenitor cells to differentiate, an antagonist of PTP HSC might act as a pan-inducer of myeloid, erythroid and lymphoid production. This use of PTP HSC antagonists may obviate or decrease the need for the use of stem cell growth factors.

Further details of the invention are illsutrated in the following non-limiting examples.

### Example 1

### Identification and cloning of murine PTP HSC

### A. Materials and Methods

**Isolation of embryonic Iin**^{**lo**}**CD34**^{**hi**}**Sca**^{**hi**} **hematopoietic stem cells.** Yolk sacs or embryos were dissected from timed pregnant females at day 10.5. Fetal livers were isolated from day 13.5-14 embryos. Yolk sac and embryonic tissues were dissociated with 1% collagenase in RPMI medium at 37°C for 15 minutes. Cells were further dissociated by two passages through a 16 gauge needle. Fetal liver was only dissociated by passage through a 16 guage needle. Adherent cells were attached to plastic by overnight incubation, after which the non adherent hematopoietic cells were incubated with a lineage cocktail of antibodies (1 µg each of TER 119, Gr-1, Ly-1, transferrin receptor and B220) for 1 hr on ice. Cells were washed, and the lineage positive cells were depleted using magnetic beads and a Miltenyi column. Lineage negative cells were pelleted, resuspended in 2% FCS, PBS and incubated with rabbit anti-murine CD34 antibody (Baumhueter *et al*., Science 262, 436-38 [1993]) on ice for 1 hr. Cells were washed three times in 2% FCS, PBS, resuspended in the same buffer and incubated with donkey, anti-rabbit FITC conjugated antibody and, in some cases, PE conjugated anti Sca antibody for 1 hr on ice. The cells were washed five times with 2% FCS, PBS, and than isolated by cell sorting on an ELITE cell sorter.

**PCR analysis of mRNA isolated from lin**^{**lo**}**CD34**^{**hi**}**Sca**^{**hi**} **hematopoietic stem cells.** Messenger RNA was isolated from the Lin^{Lo}CD34^{hi}Sca^{hi} fraction of fetal yolk-sac hematopoietic cells (Micro-FastTrack, lnVitrogene). Poly A+ RNA was reverse transcribed with random hexamers (Promega) and Moloney murine Leukemia virus revere transcriptase (Superscript II, GIBCO BRL). 1/4 of this cDNA was amplified by PCR using degenerate mixed oligonucleotides primers. Sense and antisense primers corresponding to the concensus PTP amino acid sequences ^{H}/_{D}FWRM^{I}/_{V}W (5'-A^{C}/_{T}TT^{C}/_{T}TGG^{A}/_{C}GIATG^{A}/_{G}TITGG-3') (SEQ. ID. NO: 14, where the degenerate positions are designated by "N") and WPD^{F}/_{H}GVP (5'-GGIAC^{G}/ _{A}^{T}/_{A} ^{G}/_{A} ^{G}/_{A}TCIGGCCA-3') (SEQ. ID. NO: 15, wherein the degenerate positions are designated by "N") respectively were used. PCR were carry out in 1X Taq DNA polymerase buffer (GIBCO BRL) plus 0.2 mM of each dNTP, 10% DMSO and 5 units Taq polymerase (GIBCO BRL) for 25 cycles of 94° C for 1 minute, 55° C for 1 min and 72° C for 1 minute. The PCR products were treated with Klenow enzyme (New England Biolabs) at 30°C for 30 minutes, cloned into Smal site of pRK-5 (EP 307,247, published March 15, 1989) plasmid, and subsequently sequenced (Sequenase, USB).

**cDNA and genomic cloning.** Adapter-linked double strain cDNA was prepared from A+ RNA of day-10 murine embryos (Marathon-ready cDNA synthesize kit, Clontech) using either random hexamer or oligo dT primer. Full-length cDNA was isolated by 5' or 3' rapid amplification of cDNA ends (RACE) of the marathon-ready cDNAs. Genomic clones encoding the PTP HSC gene were isolated using standard techniques. The plaque purified lambda phage DNA was digested with Not 1, and the insert fragment was directly cloned without purification into Not 1 digested Bluescript. Exons were mapped using a combination of restriction digestion and southern blotting as well as DNA sequencing using custom primers.

**Bacterial expression of the PTP.** cDNA sequences encode amino acid 8 to 323 containing the phosphatase domain were obtained by PCR using sense oligomer 5'-CACGGTCGACGGTGAGGAGCTTCTTTGAGCAGCTGGAGG-3' (SEQ. ID. NO: 3), and antisense oligomer 5'-GTTGCGGCCGCGATTGGAGCGCAGTTCTCCTTGAGGTTCTGG-3' (SEQ. ID. NO: 4). The PCR fragment was treated with Sail and Notl restriction enzyme and cloned into Sall and Notl digested pGEX-4T-1 plasmid (Pharmacia). Fusion protein was affinity purified using a glutathione sepharose column (Pharmacia). Tyrosine phosphatase assays on the GST-fusion protein were carried out following the manufacture's procedure using two different tyrosine phosphorylated peptides from a tyrosine phosphatase assay kit (Boehringer Mannheim).

**Quantitative PCR analysis of RNA isolated from hematopoietic cells.** cDNA was made from ⁺ RNA by reverse transcription (RT) with random hexamer. PCR was then used to amplified quantitatively PTP HSC cDNA and, as an internal standard, triosephosphate isomerase (TPI) cDNA. For each PCR, 6 ul of the 20 ul RT reaction was brought to 50 ul so as to contain 0.3 mM of dNTPs, 4µCi of ³²P dATP (3,00OCi/mmol, Amersham), 100 pmol of each of the four primers, and 5 units of Taq DNA polymerase (GIBCO BRL). Seventeen PCR cycles of 94° C for 50 seconds, 55° C for 50 seconds, and 70° C for 70 seconds. One-tenth of each PCR samples was electrophoresed in a 6% polyacrylamide gel, and the PCR products were quantitate by phosphorimaging (Fuji). Conditions for accurate quantitation of either PTP HSC or TPI were assessed in experiments that used serial dilutions of a standard preparation of A⁺ RNA from 32D cells to determine for each primer pair the times of primer annealing and primer extension and the cycles that provided for a linear correlation between the amount of template RNA and the PCR product. Under the PCR conditions ultimately chosen, certain amount of sample RNA was analyzed simultaneously with serial dilutions of the standard RNA and a reverse transcriptase minus control.

**Northern blot analysis of tissues and cell lines.** A Sall-Notl 1.3 kb PTP HSC cDNA fragment was used to probe murine multi-tissue northern blot (Clontech). The same northern blot was used with various other probes, all of which demonstrated detectable, undegraded transcripts.

**PCR primer pairs** 5' RACE primers: antisense primer 5'-CCTGGAGGGTCCTGAGAGTGATGTCTGCATTCAGTG-3' (SEQ. ID. NO: 5), 5'-CCTCTTGGAGCAGGGAAAGGATGACTCTTGTCTC-3' (SEQ. ID. NO: 6), 5'-CAGCTGCTCCAAGAAGCTCCTCACCAAGTC-3' (SEQ. ID. NO: 7). Sense primer: AP1 and AP2 (Clontech).
3'RACE primers: sense primer 5'-GGTAGAGGTGGGCAGGGTGAAGTGTTCTCGC-3' (SEQ. ID. NO: 8), 5'-CACTGAATGCAGACATCACTCTCAGGACCCTCCAGG-3' (SEQ. ID. NO: 9), 5'-GAGACAAGAGTCATCCTTTCCCTGCTCCAAGAGG-3' (SEQ. ID. NO: 10). Antisense primer: AP1 and AP2 (Clontech).
Quantitative RT-PCR primers: PTP HSC sense primer 5'-CACTGAATGCAGACATCACTCTCAGGACCCTCCAGG-3' (SEQ. ID. NO: 9), antisense primer 5'-GAATGGTAACCTGGAGGGTCCTGAG-3' (SEQ. ID. NO: 11). TPI sense primer 5'-GAGAAGGTCGTGTTCGAG (SEQ. ID. NO: 12), antisense primer 5'-GTGTACTTCCTGTGCCTG-3' (SEQ. ID. NO: 13).

### B. cDNA cloning of PTPs from Hematopoietic Stem Cells

In order to analyze PTPs potentially involved with the maintenance of the hematopoietic stem cell, we isolated a highly purified population of these cells from either the murine10.5 day yolk sac or embryo. Previously, we showed that both progenitor activity as well as stromal cell repopulating activity were found in the CD34^{hi} fraction of these embryonic cells [3] (C. Fennie and L. Lasky-unpublished observations). In addition, others have shown that the murine CD34^{hi} population isolated from bone marrow (Krause *et al*., Blood 84(3), 691-701 [1994]), or fetal liver (Ziegler *et al*., Blood 84, 2422-2450 [1994]) contains stem cells capable of reconstituting lethally irradiated animals. In order to isolate a more highly purified fraction of these progenitor cells, we included a lineage depletion step as well as a positive selection step with the Sca antibody (Uchida *et al*., Blood 83(12), 3758-3779 [1994]), in addition to the CD34 antibody. These morphologically primitive hematopoietic cells show a higher degree of stromal cell repopulating ability as well as cobblestone formation as compared to the previously described CD34^{hi} progenitor cells, and we are currently investigating their in vivo repopulating activity (C.Fennie and L. Lasky-unpublished observations). Previous investigators have shown that the lin ^{lo}Sca^{hi} fraction of bone marrow hematopoietic cells has a high level of repopulating activity (Sprangrude *et al*., Science 241, 58-62 [1988]). Thus, it is likely that the lin^{lo}CD34^{hi} Sca^{hi} cells isolated from the early embryo contain self renewing hematopoietic stem cells (Uchida *et al*., *supra*; Krause *et al*., *supra*; Ziegler *et al*., *supra*.

Consensus PCR using primers derived from two highly conserved regions of the PTP phosphatase domain resulted in the cloning and sequencing of ∼ 70 PCR fragments. As shown in Table 1, a diversity of known receptor and non-receptor PTPs were detected in this fraction of these progenitor cells, and many of these PTPs have not previously been described in the hematopoietic stem cell compartment. Two novel PTPs (referred to in the table as PTP 38 and PTP 49) were also isolated. One is a receptor PTP which is related to the homotypically interacting µ, κ and LAR family and is the subject of a patent application filed concurrently herewith. The second PTP was found to be most homologous to two previously described non-receptor PTPs, murine PTP PEP (Matthews *et al*., Mol. Cell Biol. 12(5), 2396-2405 [1992]) and murine/human PTP PEST (Takekawa *et al*., Biochem. Biophvs. Res. Commun. 189(2), 1223-1230 [1992]; Yang *et al*., J. Biol. Chem. 268(23) 17650 [1993]; and Charest *et al*., Biochem J. 308(2), 425-432 [ 1995]), both of which contain a region that is very high in proline, glutamate, serine and threonine (the "PEST" domain). One of these PTPs, PEP, has been demonstrated to be localized to the nucleus (Flores *et al*., *supra)* (see below), so it appeared that the novel PTP fragment may have been a new member of this potentially nuclear- localized PTP family.

Initial PCR and northern analyses with the PTP fragment revealed that the transcript encoding this enzyme is extremely rare in embryonic and adult tissues. Thus, the full length cDNA was cloned using the RACE procedure and RNA isolated from day 10 embryos. Because the RACE cloning of the 5 prime region was particularly difficult, the final 5 prime sequence was confirmed using the genomic clone encoding this PTP. As can be seen in figure 1, this transcript encodes an open reading frame of 453 amino acids specifying a protein of molecular weight 50,253 daltons. Homology searches revealed that the region encoding amino acids 25-290 were highly homologous to a variety of PTPs, with the highest degree of homology with murine PTP PEP (Matthews *et al*., *supra*) and murine/human PTP PEST (Takekawa *et al*., *supra*; Yang *et al*., *supra*; and Charest *et al*., *supra)* (figure 2). Interestingly, PTP PEP has also been found to be expressed in mature hematopoietic cells (Matthews *et al*., *supra,* Flores *et al*., *supra)* although human and murine PTP PEST appear to have a more generalized expression pattern (Yang *et al*., *supra*; Charest *et al*., *supra).* As has been shown in these two previously described PTPs, the novel PTP reported here contains a region 3 prime of the PTP domain which is very rich in proline, serine, and threonine (~29%) (boxed residues in figure 1). This region lacks other significant homology with PTPs PEP and PEST, and it is also much shorter in the novel PTP described here. Finally, a short region of 20 amino acids at the very carboxy terminus of the protein is highly homologous to similar carboxy-terminal regions in PTPs PEP and PEST (figure 2). This region is rich in basic residues and the homologous area in PTP PEP has been shown to be involved with the localization of this enzyme to the nucleus (Flores *et al*., *supra).* However, this region also contains two negatively charged residues (arrowheads in figure 2), so it is likely that this novel PTP is a cytoplasmically localized enzyme, as has been demonstrated for PTP PEST (Charest *et al*., *supra).* Finally, the novel PTP described here contains a serine residue at position 37 (shown started in figure 2) which is conserved in all three members of this family and which has been shown to be phosphorylated in PTP PEST by protein kinases C and A (Garton and Tonks, EMBO J. 13(16), 3763-71 [1994]). Interestingly, increased phosphorylation at this site is inhibitory to the PTPase activity of this PTP (Banville *et al*., Genomics 27(1), 165-173 [1995]). In summary, the novel PTP described here appears to be a new member of a family of non-receptor PTPs which contain P, S and T rich regions (figure 3). In addition, all three of these PTPs contain a homologous carboxy-terminal region which has been shown to function as a nuclear localization signal for one of the family members (PTP PEP), although the murine PEST enzyme has been found to localize to the cytoplasm.

Previous analyses of the genomic structures of other PTPs suggested that these enzymes were constructed from genes containing a large number of introns. This appears to be the case for the novel PTP described here as well. As can be seen from figure 4, the hematopoietic progenitor cell PTP gene is subdivided by 14 introns. Analysis of the intronic structure of this novel PTP as compared with that found for other PTPs suggests that the novel progenitor cell enzyme is divided into a comparable number of coding exons (for example, Banville *et al*., *supra).* In addition, as described below, there appears to be at least one other smaller transcript, as well as a heterogeneous collection of large transcripts, suggesting that alternate splicing may occur in this gene. Finally, chromosomal localization studies have demonstrated that the gene encoding the human form of this PTP is found on chromosome 14 (D. Dowbenko and L. Lasky, unpublished data).

While the sequence of the N-terminal PTP domain contained many of the conserved amino acids found to be critical for substrate recognition and tyrosine dephosphorylation (Jia *et al*., *supra),* it was important to demonstrate that this sequence indeed encoded an active PTP domain . To this end we produced a construct using the glutathione-S-transferase (GST) fusion system which contained the entire PTP-homologous region derived from the novel cDNA clone. The protein was isolated from induced cultures of bacteria, and it was tested for the dephosphorylation of tyrosine using two different phosphorylated peptides (see materials and methods). As can be seen from figure 5, the isolated GST-PTP domain fusion protein had a very high level of PTP activity, with significant dephosphorylation at only 20 picograms of enzyme per reaction, which was partially sensitive to inhibition by orthovanadate. The only partial inhibition of enzyme activity by orthovanadate was likely due to the high level of activity as well as insufficient levels of the inhibitor. These data indicate that this hematopoietic progenitor cell PTP is an active tyrosine phosphatase.

### C. Expression of the progenitor cell PTP transcript

The isolation of the novel PTP from the lin^{lo}CD34^{hi}sca^{hi} population of hematopoietic stem cells suggested that this PTP might be specific for very early progenitor cells. As figure 6A illustrates, quantitative PCR comparing the levels of the transcript encoding this PTP in the lin^{lo}CD34^{hi}sca^{hi}, a largely undifferentiated population containing hematopoietic stem cells (Spangrude *et al*., *supra;* Krause *et al*., *supra;* Zeigler *et al*., Blood 84(8), 2422-2430 [1994]), versus the lin^{lo}CD34^{hi}sca^{lo} population, a more differentiated cell population (Spangrude *et al*., *supra),* containing committed progenitors, demonstrated that there was an approximately 10 fold lower level of the transcript in the more differentiated sca^{lo} cells. In order to examine if this downregulation continued as differentiation progressed, quantitative PCR was performed using RNA isolated from suspension cultures of lin^{lo}CD34^{hi}sca^{hi} cells that were exposed to IL-1, IL-3, EPO and GM-CSF for various periods of time in the absence of stromal cells. Analysis of cell numbers, together with Wright-Giemsa staining of the cultures, revealed that the undifferentiated lin^{lo}CD34^{hi}sca^{hi} cell population dramatically expanded in the presence of these growth and differentiation factors and also metamorphosed along the myeloid pathway to ultimately give rise to cultures that contained predominately macrophages after 14 days (data not shown). As figure 6B illustrates, the transcript encoding the novel PTP disappears as the cells replicate and develop, and it is completely absent after approximately 7 days in culture. These data are consistent with a role for this PTP in early stem or progenitor cells, but not in the mature, committed cell populations.

The potential importance of this PTP specifically to the hematopoietic system is illustrated in figure 7A where northern blot analyses of various tissues and cell lines are shown. As can be seen from this figure, the transcript appears to be undetectable in the embryonic samples, and it is expressed at exceedingly low levels in adult lung and kidney. Thus, while there are clearly hematopoietic stem cells in the embryo, they must be so rare as to not allow for the direct detection of the transcript encoding the novel PTP. Particularly interesting is the lack of a signal in the RNA isolated from the adult spleen, a hematopoietic compartment that contains predominately mature, differentiated hematopoietic cells and which was previously shown to express PTP PEP (Matthews *et al*., *supra).* The very faint transcripts detected in the lung have been confirmed by non-quantitative PCR analysis (J. Cheng and L. Lasky-unpublished data). However, the transcripts in the lung are very rare and may be aberrant, since screening of an adult lung library (1x10⁶ clones) resulted in only two positive isolates, both of which contained introns (J. Cheng and L. Lasky-unpublished observations).

The lack of detectable signal in most tissues of the adult and embryo, coupled with the identification of the transcript in the highly purified stem cell population, but not in the differentiated hematopoietic cells, suggested that this PTP might be expressed in hematopoietic progenitor cell lines. As figure 7B illustrates, the transcripts encoding this novel PTP are easily detectable in the three different murine hematopoietic progenitor cell lines tested by both northern and PCR analyses. In all three cases, these lines represent relatively undifferentiated precursors of mature hematopoietic cells, although they are certainly not self-renewing stem cells. The cells appear to encode two major transcripts, in addition to a diversity of minor transcripts. One major transcript is an ∼1.8 kB RNA that corresponds to the cDNA clone described above, while the other encodes a ~0.7 kB RNA that remains to be characterized. However, it is likely that this smaller transcript is due to alternative splicing, since, as described above, the gene encoding this PTP is divided into a large number of exons (Figure 4). Figure 7C illustrates that the PTP HSC transcript is undetectable by PCR in a differentiated T cell clone, a result which is again consistent with the downregulation of this PTP in differentiated cells. Finally, PCR analysis of various human cell lines using the murine primer pair revealed expression of a similarly sized fragment in human CMK progenitor cells, and the sequence of this PCR fragment revealed that the human homologue is highly conserved with the murine PTP (J. Cheng, Kai Wu and L. Lasky-unpublished results). In summary, the novel PTP described here appears to be expressed predominately in very early hematopoietic progenitor cells, consistent with a potential role in the regulation of the differentiation state of these cells.

### D. Discussion

The ability of the hematopoietic stem cell to self renew in the absence of differentiation is an important factor which allows for this cell to provide a large number of progeny throughout the lifetime of the organism. The maintenance of the undifferentiated state must occur at the same time as the stem cell replicates, since this cell type must be continually replenished. Thus, there must be specific mechanisms that decrease some aspects of cellular activation, such as differentiation, while not affecting others, such as division. Because tyrosine phosphorylation is a critical aspect of cellular activation, based upon the results disclosed herein, it is likely that distinctive mechanisms which regulate tyrosine phosphorylation are involved with the maintenance of the self renewing stem cell. Such specificity can be accomplished in part by the expression of the appropriate growth factors by the hematopoietic cell stroma. However, another means by which such regulation can occur is by the dephosphorylation of a subset of tyrosine phosphorylated proteins. One mechanism that would allow for specific dephosphorylation is via PTPs which recognize only a fraction of the tyrosine phosphorylated proteins in the cell. Thus, the analysis of PTPs expressed by hematopoietic stem cells might further our understanding of the mechanisms by which stem cell self renewal is attained. The non-receptor PTP described in the present application has some of the features that might be expected for a regulator of stem cell differentiation.

Several aspects of this novel PTP, which is referred to throughout the specification and claims as the PTP of hematopoietic stem cells or PTP HSC, are consistent with a role in the regulation of aspects of early hematopoietic progenitor cell biology. First, the specific expression of the transcript in very early hematopoietic progenitor cells, together with the down-regulation of the message as the cel ls differentiate, is compatible with a role for this enzyme in physiological aspects of the less differentiated stem cell. While little is understood regarding the regulation of genes in very early hematopoietic progenitor cells, the apparently unique expression of this gene predominately in these comparatively undifferentiated cells suggests that novel mechanisms of transcriptional regulation might be utilized in the control of this locus (Orkin, Curr. Opin. Cell Biol. 7(6), 870-877 [1995]). In addition, the predominate lack of expression of this PTP in most adult tissues, with the exception of extremely low levels in the lung and the kidney, is also consistent with a role for this enzyme specifically within the hematopoietic progenitor cell compartment. This is in stark contrast to the expression of PTP PEP, which is found in the lymphoid compartment (Takekawa *et al*., *supra*), and PTP PEST, which is apparently ubiquitously expressed in a number of cell lines and tissues (Yang *et al*., *supra*). Second, the PTP domain can be thought of as a moderator of cell activation by virtue of its ability to dephosphorylate tyrosine residues. Tyrosine phosphorylation can either up- or down-regulate the activities of various proteins (Fantl *et al*., *supra*), so that the PTP HSC might activate or inhibit a specific subset of tyrosine phosphorylated proteins. In a cell that requires a down-regulation of differentiation, this type of specific modulation would allow for the control of the phosphotyrosine levels of proteins activated by various growth factors produced by the hematopoietic stroma. Together, these data are compatible with a function for this enzyme in the modulation of development of the stem cell that is induced by the various growth factors produced by the hematopoietic microenvironment.

The hypothesis that PTPs such as PTP HSC are involved with the maintenance of an undifferentiated state in the hematopoietic stem cell suggests possibilities regarding the substrates recognized by this type of PTP. Several of the substrates for the PTPs have been previously characterized. For example, the alpha PTP, a receptor PTP, has been found to regulate the levels of src tyrosine phosphorylation which results in differentiation of neuronal progenitor cells. Lar, as well as CD45, are apparently involved with the regulation of the tyrosine phosphorylation levels of the insulin receptor (Kulas *et al*., J. Biol. Chem.. 271(2), 748-754 (1996); Kulas *et al*., J. Biol. Chem. 271(2), 755-760 [ 1996]). From the standpoint of hematopoiesis, the SH 2 domain containing PTP 1 C phosphatase has been shown to be critically involved with the regulation of myeloid development in the motheaten mouse as well as with the activation state of the EPO receptor (Schulz *et al*., *supra*; McCulloch (Klingmuller *et al*., *supra).* Finally, another SH2-containing PTP, PTP I D has been found to positively regulate the activity of the prolactin receptor (Ali *et al*., EMBO J. 15(1), 135-142 [1996]). These examples, among others, are consistent with a role for cytoplasmically-localized PTP domains in the regulation of a variety of cellular processes. However, the nature of the substrates recognized by the rarer nuclear PTP family is unknown. The dual specificity (i.e. tyrosine and serine/threonine dephosphorylation) phosphatase encoded by the cdc25 locus is a nuclear enzyme that is critical for the regulation of mitosis (Gautier *et al*., Cell 67(1), 197-2 It [1991]). In addition, PAC-1, another nuclear localized PTP, appears to be involved with the regulation of the mitogen activated protein kinases. A recently described dual specificity phosphatase, TYP 1, related to the vaccinia virus VH 1 phosphatase, appears to be involved with the regulation of both the ERK and JNK family of mitogen activated protein kinases (King *et al*., Oncogene 11, 2553-2563 [1995]). These data suggest that several currently described phosphatases appear to play roles in the regulation of tyrosine phosphorylated nuclear proteins.

Another possible substrate for both the nuclear and cytoplasmic PTP enzymes are the STAT proteins. These transcriptional activators encompass a family of at least 6 different members, all of which are activated by the JAK tyrosine kinases (Darnell *et al*., Science 264(5164), 141501421 [1994]; ihle *et al*., Annu. Rev. Immunol. 13, 369-398 [1995]). JAK phosphorylation is stimulated by the formation of receptor complexes that are stimulated by the binding of various hematopoietic and other growth factor-like molecules (Darnell *et al*., *supra).* The phosphorylated STAT proteins than dimerize, migrate to the nucleus and bind specifically to various DNA elements that regulate the transcription of growth and differentiation genes (Shuai *et al*., Science 261(5129). 1744-1746 [1993]; Heim *et al*., Science 267(5202), 1347-49 [1995]). Thus, because these transcription factors are linked with the activation of hematopoietic differentiation factors, they provide appealing targets for negative regulation in hematopoietic stem cells. The absolute requirement for tyrosine phosphorylation of these transcriptional activators thus suggests that the novel PTP reported here could regulate STAT activation via dephosphorylation of tyrosine residues. In this manner, the upregulation of genes specific to the differentiated state could be inhibited by the dephosphorylation of one or more activated STAT molecules. This hypothesis is especially appealing in the case of the hematopoietic stem cells. In this case, the activation of the STAT proteins by the binding of various hematopoietic growth and differentiation factors, a state which would induce terminal differentiation, could be downregulated by a stem cell specific PTP such as PTP HSC. If this hypothesis is correct, the manner by which specific STAT dephosphorylation occurs must be investigated. However, it is possible that the proline, serine, threonine rich domain ofPTP HSC might function to bind to only a subset of STATs.

Finally, recent data have shown that PTP PEST can associate with the p52^{shc} and p66^{shc} SH2-containing adaptorproteins in a protein kinase C dependent fashion (Habib *et al*., J. Biol. Chem. 269(41 ), 25243-25246 [1994]). This association was through an interaction between the N-terminal region of SHC and the carboxy-terminal P,S,T rich region of the PTP PEST. The fact that this association was enhanced by protein kinase C suggested that serine or threonine phosphorylation might be involved, and a serine in the P,S,T rich region of PTP PEST is known to be phosphorylated by protein kinase C (Garton and Tonka, *supra*). Interestingly, carbachol, an activator of G protein coupled signaling, was also able to stimulate this association, suggesting that PTP PEST may be involved with the cross talk between G coupled and tyrosine kinase pathways. Because of the similarity of PTP HSC to PTP PEST, we suggest that the novel hematopoietic cell PTP of the present invention may also interact with SHC, and we are currently examining this possibility using the yeast two hybrid system.

In summary, the data disclosed in this example suggest that hematopoietic stem/progenitor cells specifically express a PTP which appears to be downregulated as the cells differentiate. The PTP seems to be predominately specific to hematopoietic progenitor cells, suggesting an important role in the development of this cell compartment. However, while these data are potentially important, a number of studies remain to be accomplished. Thus, the possibility that the STATs are substrates for this enzyme, the possible interaction of the enzyme with SHC, the constitutive expression of the enzyme in transfected cells and in transgenic animals, and the effects of null mutations at this locus in vivo may provide for further insights into the mechanisms by which stem cell self renewal is regulated.

### Example 2

### Cloning of a human PTP HSC

Two oligonucleotides (sense: 5'ACTTGGTGAGGAGCTTCTTGGAGCAGCTGGAGG3' (SEQ. ID. NO: 20), and antisense: 5'GGAATGTAACCTGGAGGGTCCTGA3' (SEQ. ID. NO: 21)) were used as PCR primers with reverse transcribed RNA isolated from human CMK hematopoietic progenitor cells. The conditions for PCR were identical to those described in Example 1 for the isolation of the PCR fragment encoding murine PTP HSC. The PCR fragment was subcloned into pBS (Bluescript) plasmid, and the DNA sequence was determined as described for the murine sequence in Example 1. The partial nucleotide sequence and deduced amino acid sequence of the human PTP HSC are shown in Figure 8.

### Example 3

### Expression of the murine and human PTP HSC

The native murine PTP HSC polypeptides are expressed in mammalian cells using standard techniques. Briefly, a DNA fragment encoding the entire PTP HSC is ligated into an expression vector (e.g. PRK5). The expression vector is then transfected into mammalian cells (e.g. embryonic kidney 292 cells), and the protein expression is determined using a monoclonal or polyclonal antibody directed against the native PTP HSC to be expressed.

**Table 1**

| PTPs expressed in lin^{lo} CD34^{hi} hematopoietic progenitor cells | | |
|---|---|---|
| Name (GenBank) | Frequency (%) | Type |
| MMPRTYPHA | ~27 | receptor, single catalytic domain |
| MUSC57B16A | ~17 | cytoplasmic, band 4.1 homology |
| MUSHCPA | ~14 | cytoplasmic SH2 domains, hematopoietic cells |
| MMPTPNU3 | ~11 | receptor |
| MMMPTPPES | ~4 | cytoplasmic, pst DOMAIN |
| MUSCPTP | ~4 | cytoplasmic |
| MUSPTPA | ~4 | receptor, kappa, homophilic interacting |
| MMTPBLR | ~3 | receptor, epithelial cells, membrane binding |
| RNU28356 | ~3 | cytoplasmic |
| RATOSTP | ~1 | receptor, FNIII domains |
| MUSPTPRL 10 | ~1 | cytoplasmic, band 4.1 homology |
| M60103 | ~1 | receptor, CD45 |
| PTP-38 (novel) | ~1 | cytoplasmic, PST family related |
| PTP-49 (novel) | ~1 | receptor related mu/kappa family |

## Claims

1. An isolated non-receptor protein tyrosine phosphatase of hematopoietic stem cells (PTP HSC), which
(1) is encoded by DNA hybridizing under stringent conditions to the complement of the nucleic acid of SEQ ID NO:1 or SEQ ID NO:16;
(2) is expressed in early hematopoietic stem cells or progenitor cells;
(3) its expression is downregulated in differentiated hematopoietic cells; and
(4) comprises an N-terminal tyrosine phosphatase domain, followed by a region rich in serine, threonine, and proline, and a carboxy terminal region of about 15 to 25 amino acids rich in basic amino acid residues.

2. The PTP HSC of claim 1 which is murine.

3. The PTP HSC of claim 1 which is human.

4. The PTP HSC of claim 1, which downregulates STAT activation.

5. An isolated non-receptor protein tyrosine phosphatase of hematopoietic stem cells (PTP HSC) selected from the group consisting of:
(1) a protein comprising the amino acid sequence shown in SEQ ID NO:2;
(2) a protein comprising the amino acid sequence shown in SEQ ID NO:17; and
(3) a further mammalian homologue of protein (1) or protein (2) which is expressed in early hematopoietic stem cells or progenitor cells, and the expression of which is downregulated in differentiated hematopoietic cells of another mammalian species, comprising an active N-terminal tyrosine phosphatase domain, retaining a serine residue at a position corresponding to amino acid position 37 in SEQ ID NO:2, a region rich in serine, threonine, and proline, retaining an active site cysteine residue at a position corresponding to amino acid position 229 in SEQ ID NO:2, and a carboxyterminal region showing at least about 80% sequence identity with the amino acid sequence between positions 430 and 451 in SEQ ID NO:2.

6. An isolated nucleic acid molecule encoding the PTP HSC of claim 1 or claim 5.

7. A vector comprising the nucleic acid molecule of claim 6 operably linked to control sequences recognized by a host cell transformed with the vector.

8. A host cell in cell culture transformed with the vector of claim 7.

9. An antibody capable of specific binding to the PTP HSC of claim 1 or claim 5.

10. The antibody of claim 9 which is an antagonist of PTP HSC.

11. A hybridoma cell line producing an antibody of claim 9 or claim 10.

12. An assay for identifying an antagonist or agonist of a PTP HSC of claim 1 or claim 5, which comprises contacting the phosphatase domain of said PTP HSC with a candidate antagonist or agonist, and monitoring the ability of said phosphatase domain to dephosphorylate tyrosine residues.

13. A method for the differentiation of undifferentiated malignant hematopoietic cells in cell culture, comprising contracting said cells with an antagonist antibody of a PTP HSC according to claim 1 or claim 5.

14. The method of claim 13 wherein said cells are leukemia cells.

15. A method for the induction of differentiation of non-embryonic stem cells in cell culture, comprising contacting said cells with an antagonist antibody of a PTP HSC according to claim 1 or claim 5.

16. A method for the expansion of undifferentiated non-embryonic stem cells in cell culture, comprising (a) adding to said cell culture a PTP HSC of claim 1 or claim 5, and (b) cultivating said undifferentiated stem cells.

17. Use of an agonist antibody specifically binding a PTP HSC as defined in claim 1 or claim 5, and a hematopoietic growth factor in the manufacture of a medicament for the expansion of undifferentiated stem cells in a patient.

18. Use of an antagonist antibody of a PTP HSC according to claim 1 or claim 5, in the manufacture of a medicament for the differentiation of undifferentiated malignant hematopoietic cells.

19. The use of claim 18 wherein said cells are leukemia cells.

20. Use of an antagonist antibody of a PTP HSC according to claim 1 or claim 5, in the manufacture of a medicament for the induction of differentiation of stem cells.

21. An antagonist antibody of a PTP HSC according to claim 1 or claim 5 for use in medicine.

22. An agonist antibody specifically binding PTP HSC as defined in claim 1 or claim 5 for use in medicine.

## Patentansprüche

1. Isolierte Nichtrezeptor-Proteintyrosinphosphatase von hämopoetischen Stammzellen (PTP HSC),
(1) die von DNA, die unter stringenten Bedingungen mit dem Komplement der Nucleinsäure von SEQ ID NO:1 oder SEQ ID NO:16 hybridisiert, codiert wird;
(2) die in frühen hämopoetischen Stammzellen oder Vorläuferzellen exprimiert wird;
(3) deren Expression in differenzierten hämopoetischen Zellen herunterreguliert ist; und
(4) die eine N-terminale Tyrosinphosphatasedomäne, eine anschließende an Serin, Threonin und Prolin reiche Region und eine carboxyterminale Region von etwa 15 bis 25 Aminosäuren, die an basischen Aminosäureresten reich ist, umfasst.

2. PTP HSC nach Anspruch 1, die murin ist.

3. PTP HSC nach Anspruch 1, die human ist.

4. PTP HSC nach Anspruch 1, die die STAT-Aktivierung herunterreguliert.

5. Isolierte Nichtrezeptor-Proteintyrosinphosphatase von hämopoetischen Stammzellen (PTP HSC), die ausgewählt ist aus der Gruppe von:
(1) einem Protein, das die in SEQ ID NO:2 gezeigte Aminosäuresequenz umfasst;
(2) einem Protein, das die in SEQ ID NO:17 gezeigte Aminosäuresequenz umfasst; und
(3) einem weiteren Säugerhomologon von Protein (1) oder Protein (2), das in frühen hämopoetischen Stammzellen oder Vorläuferzellen exprimiert wird und dessen Expression in differenzierten hämopoetischen Zellen anderer Säugerarten herunterreguliert ist, das eine aktive N-terminale Tyrosinphosphatasedomäne, die einen Serinrest an einer der Aminosäureposition 37 in SEQ ID NO: 2 entsprechenden Position beibehält, eine an Serin, Threonin und Prolin reiche Region, die einen Cysteinrest eines aktiven Zentrums an einer der Aminosäureposition 229 in SEQ ID NO:2 entsprechenden Position beibehält, und eine carboxyterminale Region, die mindestens etwa 80 % Sequenzidentität mit der Aminosäuresequenz zwischen den Positionen 430 und 451 in SEQ ID NO:2 zeigt, umfasst.

6. Isoliertes Nucleinsäuremolekül mit Codierung für die PTP HSC nach Anspruch 1 oder Anspruch 5.

7. Vektor, der das Nucleinsäuremolekül nach Anspruch 6 in funktionaler Verknüpfung mit Kontrollsequenzen, die von einer mit dem Vektor transformierten Wirtszelle erkannt werden, umfasst.

8. Wirtszelle in einer Zellkultur, die mit dem Vektor nach Anspruch 7 transformiert ist.

9. Antikörper, der spezifisch an die PTP HSC nach Anspruch 1 oder Anspruch 5 binden kann.

10. Antikörper nach Anspruch 9, der ein Antagonist von PTP HSC ist.

11. Hybridomzelllinie, die einen Antikörper nach Anspruch 9 oder Anspruch 10 produziert.

12. Test zur Identifizierung eines Antagonisten oder Agonisten einer PTP HSC nach Anspruch 1 oder Anspruch 5, der das Inkontaktbringen der Phosphatasedomäne der PTP HSC mit einem Antagonist- oder Agonistkandidaten und das Überwachen der Fähigkeit der Phosphatasedomäne zur Dephosphorylierung von Tyrosinresten umfasst.

13. Verfahren zur Differenzierung undifferenzierter bösartiger hämopoetischer Zellen in Zellkultur, das das Inkontaktbringen der Zellen mit einem Antagonistantikörper einer PTP HSC nach Anspruch 1 oder Anspruch 5 umfasst.

14. Verfahren nach Anspruch 13, wobei die Zellen Leukämiezellen sind.

15. Verfahren zur Induktion der Differenzierung von nichtembryonalen Stammzellen in Zellkultur, das das Inkontaktbringen der Zellen mit einem Antagonistantikörper einer PTP HSC nach Anspruch 1 oder Anspruch 5 umfasst.

16. Verfahren zur Expansion undifferenzierter nichtembryonaler Stammzellen in Zellkultur, das (a) die Zugabe einer PTP HSC nach Anspruch 1 oder Anspruch 5 zu der Zellkultur und (b) das Kultivieren der undifferenzierten Stammzellen umfasst.

17. Verwendung eines Agonistantikörpers, der spezifisch an eine PTP HSC gemäß der Definition in Anspruch 1 oder Anspruch 5 bindet, und eines hämopoetischen Wachstumsfaktors bei der Herstellung eines Medikaments zur Expansion undifferenzierter Stammzellen bei einem Patienten.

18. Verwendung eines Antagonistantikörpers einer PTP HSC nach Anspruch 1 oder Anspruch 5 bei der Herstellung eines Medikaments zur Differenzierung undifferenzierter bösartiger hämopoetischer Zellen.

19. Verwendung nach Anspruch 18, wobei die Zellen Leukämiezellen sind.

20. Verwendung eines Antagonistantikörpers einer PTP HSC nach Anspruch 1 oder Anspruch 5 bei der Herstellung eines Medikaments zur Induktion der Differenzierung von Stammzellen.

21. Antagonistantikörper einer PTP HSC nach Anspruch 1 oder Anspruch 5 zur Verwendung in der Medizin.

22. Agonistantikörper, der spezifisch an PTP HSC gemäß der Definition in Anspruch 1 oder Anspruch 5 bindet, zur Verwendung in der Medizin.

## Revendications

1. Protéine-tyrosine phosphatase de cellules souches hématopoïétiques (PTP HSC), non réceptrice, isolée, qui
(1) est codée par une hybridation de l'ADN dans des conditions stringentes au complément de l'acide nucléique de la SEQ ID NO : 1 ou de la SEQ ID NO : 16 ;
(2) est exprimée dans des cellules souches hématopoïétiques (HSC) précoces ou dans des cellules progénitrices ; dont
(3) l'expression est régulée à la baisse dans les cellules hématopoïétiques différenciées ; et qui
(4) comprend un domaine tyrosine phosphatase N-terminal, suivi d'une région riche en sérine, thréonine et proline, et une région carboxy-terminale d'environ 15 à 25 acides aminés riches en résidus d'acides aminés basiques.

2. PTP HSC selon la revendication 1, qui est murine.

3. PTP HSC selon la revendication 1, qui est humaine.

4. PTP HSC selon la revendication 1, qui régule à la baisse l'activation des facteurs de transcription du type STAT.

5. Protéine-tyrosine phosphatase de cellules souches hématopoïétiques (PTP HSC), non réceptrice, isolée, choisie dans le groupe constitué par :
(1) une protéine comprenant la séquence d'acides aminés, représentée dans la SEQ ID NO : 2 ;
(2) une protéine comprenant la séquence d'acides aminés, représentée dans la SEQ ID NO : 17 ; et
(3) un autre homologue mammifère de la protéine (1) ou de la protéine (2) qui est exprimé dans des cellules souches hématopoïétiques précoces ou dans des cellules progénitrices, et dont l'expression est régulée à la baisse dans les cellules hématopoïétiques différenciées des autres espèces mammifères comprenant un domaine tyrosine phosphatase N-terminal, conservant un résidu sérine à une position correspondant à la position de l'acide aminé 37 dans la SEQ ID NO : 2, une région riche en sérine, thréonine et proline, conservant un résidu cystéine du site actif à une position correspondant à la position de l'acide aminé 229 dans la SEQ ID NO : 2, et une région carboxy-terminale présentant une homologie de séquence d'au moins environ 80 % avec la séquence d'acides aminés entre les positions 430 et 451 dans la SEQ ID NO : 2.

6. Molécule d'acide nucléique isolée codant les PTP HSC selon la revendication 1 ou la revendication 5.

7. Vecteur comprenant la molécule d'acide nucléique selon la revendication 6, lié fonctionnellement à des séquences de contrôle reconnues par une cellule hôte transformée avec le vecteur.

8. Cellule hôte dans une culture de cellules transformée avec le vecteur de la revendication 7.

9. Anticorps capable de se lier spécifiquement à la PTP HSC selon la revendication 1 ou la revendication 5.

10. Anticorps selon la revendication 9, qui est un antagoniste de la PTP HSC.

11. Lignée de cellules d'hybridome produisant un anticorps selon la revendication 9 ou la revendication 10.

12. Analyse afin d'identifier un antagoniste ou un agoniste d'une PTP HSC selon la revendication 1 ou la revendication 5, qui comprend la mise en contact du domaine phosphatase de ladite PTP HSC avec un candidat antagoniste ou agoniste, et la surveillance de la capacité dudit domaine phosphatase à déphosphoryler les résidus tyrosine.

13. Procédé de différenciation des cellules hématopoïétiques malignes indifférenciées dans une culture cellulaire comprenant la mise en contact desdites cellules avec un anticorps antagoniste d'une PTP HSC selon la revendication 1 ou la revendication 5.

14. Procédé selon la revendication 13, dans lequel lesdites cellules sont des cellules leucémiques.

15. Procédé permettant l'induction d'une différenciation de cellules souches non embryonnaires dans une culture cellulaire comprenant la mise en contact desdites cellules avec un anticorps antagoniste d'une PTP HSC selon la revendication 1 ou la revendication 5.

16. Procédé d'expansion de cellules souches non embryonnaires indifférenciées dans une culture cellulaire, comprenant les opérations consistant à (a) ajouter à ladite culture de cellules une PTP HSC selon la revendication 1 ou la revendication 5, et (b) à cultiver lesdites cellules souches indifférenciées.

17. Utilisation d'un anticorps agoniste liant spécifiquement une PTP HSC telle que définie dans la revendication 1 ou la revendication 5, et un facteur de croissance hématopoïétique, dans la fabrication d'un médicament en vue de l'expansion des cellùles souches indifférenciées chez un patient.

18. Utilisation d'un anticorps antagoniste d'une PTP HSC selon la revendication 1 ou la revendication 5, dans la fabrication d'un médicament en vue de la différenciation des cellules hématopoïétiques malignes indifférenciées.

19. Utilisation selon la revendication 18, dans laquelle lesdites cellules sont des cellules leucémiques.

20. Utilisation d'un anticorps antagoniste de la PTP HSC selon la revendication 1 ou la revendication 5, dans la fabrication d'un médicament en vue de l'induction de la différentiation des cellules souches.

21. Anticorps antagoniste de la PTP HSC selon la revendication 1 ou la revendication 5, pour une utilisation en médecine.

22. Anticorps agoniste se liant spécifiquement à une PTP HSC telle que définie dans la revendication 1 ou la revendication 5, pour une utilisation en médecine.
